(19) Europäisches Patentamt
European Patent Office
Office européen des brevets

(11) EP 1 980 619 A1

(12) **EUROPÄISCHE PATENTANMELDUNG**

(43) Veröffentlichungstag:
**15.10.2008 Patentblatt 2008/42**

(51) Int Cl.:
***C12N 15/82** (2006.01)* ***C08B 30/04** (2006.01)*

(21) Anmeldenummer: **07090072.5**

(22) Anmeldetag: **10.04.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(71) Anmelder: **Bayer CropScience Aktiengesellschaft**
**40789 Monheim (DE)**

(72) Erfinder:
- **Frohberg, Claus**
  **14532 Kleinmachnow (DE)**
- **Essigmann, Bernd**
  **12157 Berlin (DE)**
- **Soyka, Stephan**
  **10823 Berlin (DE)**

(74) Vertreter: **Quanz, Martin**
**Bayer BioScience GmbH**
**Hermannswerder 20a**
**D-14473 Potsdam (DE)**

(54) **Verfahren zur Herstellung einer resistenten Stärke**

(57) Die vorliegende Erfindung betrifft Verfahren zur Herstellung von modifizierter Stärke mittels genetisch modifizierter Pflanzen, die ein fremdes Nucleinsäuremolkül codierend ein Protein mit der Aktivität einer Glutamin: Fructose-6-Phosphat Amidotransferase (GFAT) aufweisen. Weiterhin betrifft die vorliegende Erfindung Stärke erhältlich aus besagten Pflanzen, sowie Nahrungsmittel, enthaltend besagte Stärke oder Teile besagter Pflanzen.

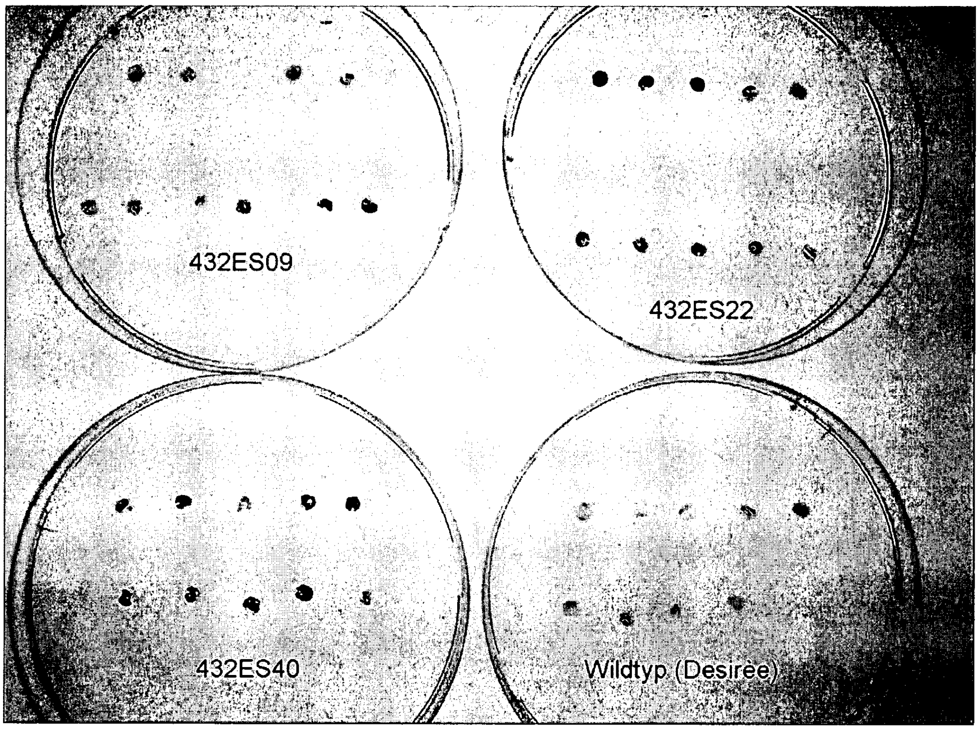


**Fig. 1**

EP 1 980 619 A1

## Beschreibung

Die vorliegende Erfindung betrifft Verfahren zur Herstellung von modifizierter Stärke mittels genetisch modifizierter Pflanzen, die ein fremdes Nucleinsäuremolkül codierend ein Protein mit der Aktivität einer Glutamin:Fructose-6-Phosphat Amidotransferase (GFAT) aufweisen. Weiterhin betrifft die vorliegende Erfindung Stärke erhältlich aus besagten Pflanzen, sowie Nahrungsmittel, enthaltend besagte Stärke oder Teile besagter Pflanzen.

Das Polysaccharid Stärke ist aus chemisch einheitlichen Grundbausteinen, den Glucosemolekülen, aufgebaut, stellt jedoch ein komplexes Gemisch unterschiedlicher Molekülformen dar, die Unterschiede hinsichtlich des Polymerisations- und des Verzweigungsgrades aufweisen und sich somit in ihren physikalisch-chemischen Eigenschaften stark voneinander unterscheiden. Man differenziert zwischen Amylosestärke, einem im wesentlichen unverzweigten Polymer aus alpha-1,4-glycosidisch verknüpften Glucoseeinheiten, und der Amylopektinstärke, einem verzweigten Polymer, bei dem die Verzweigungen durch das Auftreten zusätzlicher alpha-1,6-glycosidischer Verknüpfungen zustande kommen. Ein weiterer wesentlicher Unterschied zwischen Amylose und Amylopektin liegt im Molekulargewicht. Während Amylose, je nach Herkunft der Stärke, ein Molekulargewicht von $5x10^5$ - $10^6$ Da besitzt, liegt das des Amylopektins zwischen $10^7$ und $10^8$ Da. Die beiden Makromoleküle können durch ihr Molekulargewicht und ihre unterschiedlichen physiko-chemischen Eigenschaften differenziert werden, was am einfachsten durch ihre unterschiedlichen Jodbindungseigenschaften sichtbar gemacht werden kann.

Amylose wurde lange als lineares Polymer, bestehend aus alpha-1,4-glycosidisch verknüpften alpha-D-Glucose-Monomeren, angesehen. In neueren Studien wurde jedoch die Anwesenheit von wenigen alpha-1,6-glycosidischen Verzweigungspunkten (ca. 0,1%) nachgewiesen (Hizukuri und Takagi, Carbohydr. Res. 134, (1984), 1-10; Takeda et al., Carbohydr. Res. 132, (1984), 83-92).

Der Einsatz resistenter Stärke (RS) gewinnt in der Nahrungsmittelindustrie zunehmend an Bedeutung. Stärke wird hauptsächlich im Dünndarm durch das Enzym alpha-Amylase verdaut, das die alpha-1,4-glucosidischen Bindungen der Stärke zu Zuckern hydrolysiert. Im Gegensatz dazu wird die resistente Stärke im Dünndarm nicht durch alpha-Amylasen verdaut, sondern geht in den Dickdarm über, wo sie sich ähnlich wie Ballaststoffe verhält. Aus dem Abbau von RS-haltigen Produkten bezieht der Organismus nur in geringem Umfang Energie. Diese Energiezufuhr betrifft ausschließlich den oxidativen Abbau resorbierter kurzkettiger Fettsäuren aus dem Dickdarm. Diese kurzkettigen Fettsäuren sind Endprodukte des Kohlenhydratstoffwechsels der intestinalen Mikroflora. Mit der Aufnahme RS-haltiger Nahrungsmittel werden Substrate für den Energiestoffwechsel der intestinalen Mikroflora und der Dickdarmepithelzellen bereitgestellt. Letztere sind zur Aufrechterhaltung ihrer Struktur und Funktion auf die luminale Zufuhr von kurzkettigen Fettsäuren und insbesondere von Butyrat angewiesen. Resistente Stärke ist daher nach wissenschaftlichen Erkenntnissen ein Faktor zur Verhinderung von Divertikulose und Dickdarmkrebs.

Es wird zwischen den folgenden Typen resistenter Stärke unterschieden:

RS1 Physikalisch der Verdauung unzugängliche Stärke, z.B. in ein Protein oder eine Fasermatrix eingebettete Stärke. Wenn diese physikalisch (z.B. durch Kauen) oder chemisch (z.B. durch Abbau der sie umgebenden Matrix) aufgeschlossen wird, kann sie durch die Verdauungssäfte in normaler Weise bearbeitet werden.
RS2 Unverdauliche intakte (granuläre) native Stärkekörner, z.B. ungekochte Kartoffel- oder Bananenstärke)
RS3 Unverdauliche retrogradierte Stärke, die nicht granulär ist
RS4 Unverdauliche chemisch modifizierte Stärke, z.B. durch Quervernetzung oder Veresterung (Acetylierung etc.)

Im Gegensatz zu RS 4 können die RS-Formen 1 bis 3 durch Lösung in NaOH oder Dimethylsulfoxid dem alpha-Amylase-Abbau zugänglich gemacht werden.

Zur Herstellung von resistenter Stärke wurden verschiedene Verfahren beschrieben. Die meisten dieser Verfahren betreffen die Herstellung von RS3-Stärken (EP 564893 A1; EP 688872 A1; EP 846704 A1; US5051271). Alle diese Verfahren zur Herstellung resistenter Stärke beinhalten das Dispergieren und Gelatinisieren (Verkleistern) von Stärke in großen Überschussmengen an Wasser, gefolgt von einer Retrogradation unter Verwendung von Enzymen oder Säuren. Sie beruhen auf der Auffassung, dass resistente Stärke gebildet wird, wenn die Amylosefraktion von Stärke nach der Gelatinisierung von Stärke retrogradiert wird. Man geht davon aus, dass sich die linearen Amylosemoleküle nach Gelatinisierung zu dichten, durch Wasserstoffbrückenbindungen gebundenen Doppelhelixkonfigurationen anordnen, so dass die alpha-1,4-Glucosidbindungen für alpha-Amylasen nicht mehr zugänglich sind. Die beschriebenen Verfahren zur Herstellung RS3-Stärken sind arbeitsintensiv, zeitaufwendig und können zu geringen Ausbeuten führen. Darüber hinaus kann der für die Produktion benötigte hohe Wassergehalt teure Trocknungsschritte erforderlich machen.

Granuläre Stärken des Typs RS2 mit einem hohen Gehalt an resistenter Stärke findet man vor allem bei nativen, ungekochten Wildtyp-Kartoffelstärken, die je nach Bestimmungsmethode einen RS-Gehalt zwischen 74-85 Gew.-% aufweisen (Faisant et al., Sciences des Aliments 15, (1995), 83-89; Evans and Thompson, Cereal Chemistry 81(1), (2004), 31-37).

Ein Nachteil von Stärken des Typs RS3 bzw. RS4 besteht darin, dass sie nach ihrer Herstellung Nahrungsmitteln zugegeben werden müssen. Daher können diese Stärken nur in Fertigprodukten (wie z.B. Joghurts) angereichert werden. In vom Endverbraucher selbst zubereiteter Nahrung aus frischen Produkten (wie z.B. gekochten Kartoffeln) ist es nicht möglich, den Gehalt an RS durch Zugabe dessen zu erhöhen. Werden garnuläre Stärken zu Nahrungsmitteln hinzu gegeben, so hat dieses häufig negative Auswirkungen auf die Textur des Nahrungsmittels und beim Verzehr entsteht u.a. durch die Größe der Stärkekörner ein unerwünschtes, oft sandiges Mundgefühl ("mouth feel").

Weiterhin wird zwischen schnell verdaubarer Stärke ("readily digestible starch" (RDS) und langsam verdaubarer Stärke ("Slowly digestible starch" (SDS)) unterschieden. Als RDS bezeichnet man den Anteil an Stärke, der beim Verdau mit Pankreatin innerhalb von 20 Minuten verdaubar ist, während mit SDS der Anteil an Stärke bezeichnet wird, der beim Verdau mit Pankreatin innerhalb des Zeitintervalls zwischen 20 Minuten und 120 Minuten verdaubar ist.. Als RS ("resistant starch") bezeichnet man hingegen den Anteil an Stärke, der nach 120 Minuten nicht mittels Pankreatin verdaubar ist (Englyst et al., (1992, European Journal of Clinical Nutrition 46 Suppl. 2, S33-S50).

Insbesondere Nahrungsmittel die einen hohen Anteil an RDS aufweisen führen beim Verzehr zu einem schnellen Anstieg des Blutglucosespiegels beim Konsumenten, woraufhin eine rasche Ausschüttung von Insulin erfolgt. Der fortwährende Konsum von Nahrungsmitteln mit einer hohen glykämischen Ladung, und die damit verbundene Insulinausschüttung, steht in Verdacht ein Risikofaktor bei der Entstehung von Krankheiten zu sein.

US 5,714,600 beschreibt mittels züchterischer Verfahren hergestellte Maispflanzen, die eine Stärke synthetisieren, die einen erhöhten Anteil an Amylose und einen erhöhten Anteil an RS aufweist. WO 2004 0199942 beschreibt Mutanten von Gerste Pflanzen, die eine Mutation im Gen codierend für eine Srärkesynthase II (SSII) aufweisen. Diese Pflanzen synthetisieren eine Stärke, die einen erhöhten Anteil an Amylose, einen erhöhten Anteil an beta-Glucanen und einen erhöhten Anteil an RS aufweisen.

WO 00 11192 beschreibt die endosperm-spezifische Überexpression eines Nucleinsäuremoleküls aus Mais codierend ein Protein mit der enzymatischen Aktivität einer pflanzlichen GFAT in transgenen Maispflanzen mit dem Ziel, eine modifizierte (kationische) Stärke in Pflanzen zu synthetisieren, die 2-Amino Anhydroglucose Moleküle aufweist. Der beschriebene Stoffwechselweg, welcher gemäß der Beschreibung von WO 00 11192 zum Einbau von 2-Amino-Anhydroglucose in die Stärke führen soll, umfasst u.a. den Einbau von UDP-Glucosamin mittels Stärke- und/oder Glycogensynthasen in die Stärke. In Mehl aus Endosperm der betreffenden transgenen Maispflanzen, die ein Nucleinsäuremolekül codierend ein Protein mit der enzymatischen Aktivität einer pflanzlichen GFAT translational fusioniert mit einem plastidären Signalpeptid exprimierten oder die ein Protein mit der enzymatischen Aktivität einer GFAT ohne Signalpeptid exprimierten, konnte keine kationische Stärke nachgewiesen werden.

Der vorliegenden Erfindung liegt somit die Aufgabe zu Grunde, Verfahren zur Herstellung von modifizierter Stärke, sowie Verfahren zur Herstellung der besagten Stärken, als auch Lebens- bzw. Futtermittel enthaltend besagte modifizierte Stärke, zur Verfügung zu stellen.

Ein erster Aspekt der vorliegenden Erfindung betrifft ein Verfahren zur Herstellung modifizierter Stärke in genetisch modifizierten Pflanzen, umfassend die folgenden Schritte

a) Einführung eines fremden Nucleinsäuremoleküls codierend ein Protein mit der Aktivität einer Glutamin:Fructose-6-Phosphat Amidotransferase (GFAT) in eine Pflanzenzelle
b) Regeneration einer Pflanze aus Pflanzenzellen erhalten nach Schritt a)
c) gegebenenfalls Erzeugung von weiteren Pflanzen mit Hilfe der Pflanzen nach Schritt b).
d) Extraktion von Stärke aus Pflanzen erhältlich nach Schritt b) oder c).

Es wurde überraschenderweise gefunden, dass genetisch modifizierte Pflanzen, in die ein fremdes Nucleinsäuremolekül codierend ein Protein mit der Aktivität einer Glutamin:Fructose-6-Phosphat Amidotransferase (GFAT) eingeführt wurde, eine Stärke synthetisieren, die einen höheren Anteil an resistenter Stärke aufweist im Vergleich zu Stärke, isoliert aus entsprechenden Wildtyp-Pflanzen, in die kein fremdes Nucleinsäuremolekül codierend ein Protein mit der Aktivität einer Glutamin:Fructose-6-Phosphat Amidotransferase eingeführt wurde. Solche Pflanzen bieten gegenüber Wildtyp-Pflanzen den Vorteil, dass sie eine Stärke synthetisieren, die auch nach Erhitzen (Verkleisterung der Stärke) einen erhöhten Anteil an resisitenter Stärke (RS) aufweisen. Besagte Pflanzen bzw. Teile dieser Pflanzen, die für die Herstellung von Nahrungsmitteln erhitzt werden zeichnen sich daher dadurch aus, dass sie trotz der Hitzebehandlung weiterhin einen erhöhten Gehalt an RS aufweisen. Sie weisen daher einerseits einen geringeren Kaloriengehalt auf. Andererseits enthalten solche Pflanzen einen verringerten Anteil an schnell verdaulicher Stärke (RDS), was besonders vorteilhaft ist, da eine schnelle Freisetzung größerer Mengen Glucose und dessen Absorption über das Dünndarmepithel zu einer abrupten Zunahme des Blutzuckerspiegels und damit zu einem erhöhten glykämischen Index führt. In dessen Folge kommt es zu einer Ausschüttung von Insulin (Insulin Antwort). Der fortwährende Konsum von Nahrungsmitteln mit einer hohen glykämischen Ladung, und die damit verbundene Insulinausschüttung, steht in Verdacht ein Risikofaktor bei der Entstehung von Krankheiten wie Bluthochdruck, Übergewicht, Herzkrankheiten und Diabetes Typ II zu sein.

Unter dem Begriff "fremdes Nukleinsäuremolekül" versteht man im Zusammenhang mit der vorliegenden Erfindung ein solches Molekül, das entweder natürlicherweise in entsprechenden Wildtyp-Pflanzenzellen nicht vorkommt, oder das in der konkreten räumlichen Anordnung nicht natürlicherweise in Wildtyp-Pflanzenzellen vorkommt oder das an einem Ort im Genom der Wildtyp-Pflanzenzelle lokalisiert ist, an dem es natürlicherweise nicht vorkommt.

Bevorzugt ist das fremde Nukleinsäuremolekül ein rekombinantes Molekül, das aus verschiedenen Elementen (Nucleinsäuremolekülen) besteht, deren Kombination oder spezifische räumliche Anordnung natürlicherweise in pflanzlichen Zellen nicht auftritt.

Unter dem Begriff "rekombinantes Nukleinsäuremolekül" soll im Zusammenhang mit der vorliegenden Erfindung ein Nukleinsäuremolekül verstanden werden, welches unterschiedliche Nucleinsäuremoleküle aufweist, die natürlicherweise nicht in einer Kombination vorliegen, wie sie in einem rekombinanten Nucleinsäuremolekül vorliegen. So können rekombinante Nucleinsäuremoleküle z.B., neben fremden Nucleinsäuremolekülen, die ein Protein codieren, zusätzliche Nucleinsäuresequenzen aufweisen, welche natürlicherweise nicht in Kombination mit den besagten ein Protein codierenden Nucleinsäuremolekülen vorliegen. Die genannten zusätzlichen Nucleinsäuresequenzen, die auf einem rekombinanten Nucleinsäuremolekül in Kombination mit einem Protein codierenden Nucleinsäuremolekül vorliegen, können dabei beliebige Sequenzen sein. Sie können z.B. genomische und/oder pflanzliche Nucleinsäuresequenzen darstellen. Bevorzugt handelt es sich bei genannten zusätzlichen Nucleinsäuresequenzen um regulatorische Sequenzen (Promotoren, Terminationssignale, Enhancer, Introns), besonders bevorzugt um regulatorische Sequenzen, die in pflanzlichem Gewebe aktiv sind, insbesondere bevorzugt um gewebespezifische regulatorische Sequenzen die in pflanzlichem Gewebe aktiv sind.

Methoden zur Erzeugung rekombinanter Nucleinsäuremoleküle sind dem Fachmann bekannt und umfassen gentechnische Methoden wie z.B. die Verbindung von Nucleinsäuremolekülen durch Ligation, genetische Rekombination oder die Neusynthese von Nucleinsäuremolekülen (siehe z.B. Sambrok et al., Molecular Cloning, A Laboratory Manual, 3rd edition (2001) Cold Spring Harbour Laboratory Press, Cold Spring Harbour, NY. ISBN: 0879695773; Ausubel et al., Short Protocols in Molecular Biology, John Wiley & Sons; 5th edition (2002),ISBN: 0471250929).

Ein bevorzugter Gegenstand der vorliegenden Erfindung betrifft erfindungsgemäße Verfahren zur Herstellung modifizierter Stärke worin die fremden Nucleinsäuremoleküle, codierend ein Protein mit der Aktivität einer GFAT mit regulatorischen Elementen verknüpft sind, die die Transkription in Pflanzenzellen initiieren (Promotoren). Es kann sich um homologe oder heterologe Promotoren handeln. Bei den Promotoren kann es sich um konstitutive, gewebespezifische, entwicklungsspezifische oder um durch äußere Einflüsse (z.B. nach Applikation chemischer Substanzen, durch Einwirkung abiotischer Faktoren wie Hitze und/oder Kälte, Trockenheit, Krankheitsbefall etc.) regulierte Promotoren handeln.

Generell kommt für die Expression eines fremden Nucleinsäuremoleküls jeder in pflanzlichen Zellen aktive Promotor in Frage. Geeignete Promotoren sind z.B. der Promotor der 35S RNA des Cauliflower Mosaic Virus oder der Ubiquitin-Promotor aus Mais oder der *Cestrum* YLCV Promotor (Yellow Leaf Curling Virus; WO 01 73087; Stavolone et al., 2003, Plant Mol. Biol. 53, 703-713) für eine konstitutive Expression, der Patatingen-Promotor B33 (Rocha-Sosa et al., EMBO J. 8 (1989), 23-29) für eine knollenspezifische Expression in Kartoffeln oder ein fruchtspezifischer Promotor für Tomate wie z.B. der Polygalacturonase Promotor aus Tomate (Montgomery et al., 1993, Plant Cell 5, 1049-1062) oder der E8 Promotor aus Tomate (Metha et al., 2002, Nature Biotechnol. 20(6), 613-618) oder der ACC Oxidase Promotor aus Pfirsich (Moon und Callahan, 2004, J. Experimental Botany 55 (402), 1519-1528) oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451) oder für eine endosperm-spezifische Expression der HMWG-Promotor aus Weizen, der USP-Promotor, der Phaseolinpromotor, Promotoren von Zein-Genen aus Mais (Pedersen et al., Cell 29 (1982), 1015-1026; Quatroccio et al., Plant Mol. Biol. 15 (1990), 81-93), ein Glutelin-Promotor (Leisy et al., Plant Mol. Biol. 14 (1990), 41-50; Zheng et al., Plant J. 4 (1993), 357-366; Yoshihara et al., FEBS Lett. 383 (1996), 213-218), ein Globulin Promotor (Nakase et al., 1996, Gene 170(2), 223-226), ein Prolamin Promotor (Qu und Takaiwa, 2004, Plant Biotechnology Journal 2(2), 113-125) oder ein Shrunken-1 Promotor (Werr et al., EMBO J. 4 (1985), 1373-1380). Es können jedoch auch Promotoren verwendet werden, die nur zu einem durch äußere Einflüsse determinierten Zeitpunkt aktiviert werden (siehe beispielsweise WO 9307279, deren Inhalt durch Nennung in die vorliegende Beschreibung aufgenommen wird). Von besonderem Interesse können hierbei Promotoren von heat-shock Proteinen sein, die eine einfache Induktion erlauben. Ferner können samenspezifische Promotoren verwendet werden, wie z.B. der USP-Promoter aus *Vicia* faba, der eine samenspezifische Expression in *Vicia* faba und anderen Pflanzen gewährleistet (Fiedler et al., Plant Mol. Biol. 22 (1993), 669-679; Bäumlein et al., Mol. Gen. Genet. 225 (1991), 459-467).

Auch die Verwendung von Promotoren, die im Genom von Algen infizierenden Viren vorkommen, sind für die Expression von Nucleinsäuresequenzen in Pflanzen geeignet (Mitra et al., 1994, Biochem. Biophys Res Commun 204(1), 187-194; Mitra und Higgins, 1994, Plant Mol Biol 26(1), 85-93, Van Etten et al., 2002, Arch Virol 147, 1479-1516).

Unter dem Begriff "gewebespezifisch" soll im Zusammenhang mit der vorliegenden Erfindung die überwiegende Beschränkung einer Ausprägung (z.B. die Initiation der Transkription) auf ein bestimmtes Gewebe verstanden werden.

Unter den Begriffen "Knollen-, Frucht-, oder Endospermzelle" sollen im Zusammenhang mit der vorliegenden Erfindung alle Zellen verstanden werden, die in einer Knolle, Frucht bzw. in einem Endosperm enthalten sind.

Unter dem Begriff "homologer Promotor" soll im Zusammenhang mit der vorliegenden Erfindung ein Promotor verstanden werden, der natürlicherweise in Pflanzenzellen vorkommt, die für die Durchführung erfindungsgemäßer Verfahren verwendet wurden (homolog bezüglich der Pflanzenzelle oder Pflanze) oder ein Promotor verstanden werden, der die Regulation der Expression eines Gens in dem Organismus reguliert, aus dem das betreffende für ein Protein codierende fremde Nucleinsäuremolekül, isoliert wurde (homolog bezüglich des zu exprimierenden Nucleinsäuremoleküls).

Unter dem Begriff "heterologer Promotor" soll im Zusammenhang mit der vorliegenden Erfindung ein Promotor verstanden werden, der natürlicherweise nicht in Pflanzenzellen vorkommt, die für die Durchführung erfindungsgemäßer Verfahren verwendet wurden (heterolog bezüglich der Pflanzenzelle oder Pflanze) oder ein Promotor verstanden werden, der natürlicherweise in dem Organismus, aus dem das betreffende für ein Protein codierende fremde Nucleinsäuremolekül isoliert wurde, nicht zur Regulation der Expression von besagtem fremden Nucleinsäuremolekül vorkommt (heterolog bezüglich des zu exprimierenden Nucleinsäuremoleküls).

Ferner kann eine Terminationssequenz (Polyandenylierungssignal) vorhanden sein, die der Addition eines Poly-A-Schwanzes an das Transkript dient. Dem Poly-A-Schwanz wird eine Funktion bei der Stabilisierung der Transkripte beigemessen. Derartige Elemente sind in der Literatur beschrieben (vgl. Gielen et al., EMBO J. 8 (1989), 23-29) und sind beliebig austauschbar.

Es können auch Intronsequenzen zwischen dem Promotor und der codierenden Region des fremden Nucleinsäuremoleküls vorhanden sein. Solche Intronsequenzen können zur Stabilität der Expression und zu einer erhöhten Expression in Pflanzen führen (Callis et al., 1987, Genes Devel. 1, 1183-1200; Luehrsen, and Walbot, 1991, Mol. Gen. Genet. 225, 81-93; Rethmeier et al., 1997; Plant Journal 12(4), 895-899; Rose and Beliakoff, 2000, Plant Physiol. 122 (2), 535-542; Vasil et al., 1989, Plant Physiol. 91, 1575-1579; XU et al., 2003, Science in China Series C Vol.46 No.6, 561-569). Geeignete Intronsequenzen sind beispielsweise das erste Intron des sh1-Gens aus Mais, das erste Intron des Poly-Ubiquitin Gens 1 aus Mais, das erste Intron des EPSPS Gens aus Reis oder eines der beiden ersten Introns des PAT1 Gens aus *Arabidopsis.*

Unter dem Begriff "Glutamin:Fructose-6-Phosphat Amidotransferase (GFAT)" (E.C. 2.6.1.16), in der Fachliteratur auch als Glucosamin Synthase bezeichnet, soll im Zusammenhang mit der vorliegenden Erfindung ein Protein verstanden werden, das aus den Edukten Glutamin und Fructose-6-Phosphat (Fruc-6-P) Glucosamin-6-Phosphat (GlcN-6-P) synthetisiert. Diese Katalyse folgt dabei folgendem Reaktionsschema:

Glutamin + Fruc-6-P $\rightarrow$ GlcN-6-P + Glutamat

Der Begriff "Glutamin:Fructose-6-Phosphat Amidotransferase (GFAT)" wird im Zusammenhang mit der vorliegenden Erfindung als übergreifender Begriff verwendet, der alle bekannten Isoformen umfasst.

In einem Übersichtsartikel von Milewski (2002, Biochimica et Biophysica Acta 1597, 173-193) sind strukturelle Merkmale von Proteinen mit der Aktivität einer GFAT beschrieben. Alle bekannten Proteine mit der Aktivität einer GFAT weisen in ihrer Aminosäuresequenz Bereiche mit konservierten Aminosäuresequenzen auf. Proteine mit der Aktivität einer GFAT weisen in ihrer Aminosäuresequenz eine N-terminale Glutamin-Bindedomäne und eine C-terminale Fructose-6-Phosphat-Bindedomäne auf, die durch eine Abfolge von 40 bis 90 nicht konservierten Aminosäuren voneinander getrennt vorliegen. Beide Domänen sind, wenn sie auf getrennten Aminosäuremolekülen vorliegen, aktiv. Analysen der Kristallstruktur eines Fragments umfassend die N-terminale Glutamin-Bindedomäne des Proteins mit der Aktivität einer GFAT aus *Escherichia coli* ergaben das das aktive Zentrum dieser Domäne am N-Terminus lokalisiert und die Aminosäure Cys1 an der Hydrolyse von Glutamin beteiligt ist. Die Aminosäuren Arg73 und Asp123 interagieren mit Carboxyl- und Aminogruppen des Glutamins. Diese Interaktion wird von den Aminosäuren Thr76 und His77 unterstützt. Den Aminosäuren Gly99 und Trp74 wird die Ausbildung von Wasserstoffbrückenbindungen mit der Amidgruppe des Glutamins zugeschrieben. Die Aminosäuren Asn98 und Gly99 stabilisieren die vierflächige Tasche des aktiven Zentrums. Die Aminosäuren 25 bis 29 und 73-80 bilden flexible Schleifen (loops) aus, die nach Bindung des Substrates Glutamin durch eine Konformationsänderung des Proteins an der durch ein Protein mit der Aktivität einer GFAT katalysierten Reaktion beitragen. Die Analyse der Kristallstruktur der C-terminalen Fructose-6-Phosphat-Bindedomäne des Proteins mit der Aktivität einer GFAT aus *Escherichis coli* ergab, dass diese Domäne aus zwei topologisch identischen Domänen (Aminosäuren 241 bis 424 und 425 bis 592), gefolgt von einer Domäne, die am C-terminalen Ende als irreguläre Schleife (Aminosäuren 593 bis 608) vorliegt, aufgebaut ist, jedoch nur ein aktives Zentrum besitzt. Die Aminosäuren Ser303, Ser347, Gln348, Ser349 und Thr352 sind an der Substratbindung beteiligt, während die Aminsäuren Glu488, His504 und Lys603 direkt an der Katalyse der Reaktion des Proteins mit der Aktivität einer GFAT beteiligt sind.

Insbesondere in tierischen Organismen konnten zwei unterschiedliche Isoformen von Proteinen mit der Aktivität einer GFAT nachgewiesen werden (in der Literatur bezeichnet als GFAT-1 bzw. GFAT-2). Hu et al. (2004), J. Biol. Chem. 279(29), 29988-29993) beschreiben Unterschiede der betreffenden Isoformen von Proteinen mit der Aktivität einer GFAT. Neben Unterschieden der gewebespezifischen Expression der betreffenden Isoformen, mit der Aktivität einer GFAT-1 und einer GFAT-2, konnte gezeigt werden, dass beide Isoformen durch Phosphorylierung mittels einer cAMP abhängigen Proteinkinase reguliert werden. Die Aktivität eines Proteins mit der enzymatischen Aktivität einer GFAT-1 wird durch Phosphorylierung eines konservierten Serinrestes (Serin 205 in der GFAT-1 aus Maus, GenBank Acc No.: AF334736.1) der betreffenden Aminosäuresequenz inhibiert, wohingegen die Aktivität eines Proteins mit der Aktivität einer GFAT-2 durch Phosphorylierung eines konservierten Serinrestes (Serin 202 in der GFAT-2 aus Maus, GenBank Acc No.: NM_013529) der betreffenden Aminosäuresequenz gesteigert wird. Sowohl Proteine mit der Aktivität einer GFAT-1, als auch Proteine mit der Aktivität einer GFAT-2 werden durch UDP-GlcNAc konzentrationsabhängig inhibiert, jedoch ist die Inhibition durch UDP-GlcNAc für ein Protein mit der Aktivität einer GFAT-2 geringer (maximale Verringerung der Aktivität durch UDP-GlcNAc um ca. 15%), im Vergleich zu einem Protein mit der Aktivität einer GFAT-1 (maximale Verringerung der Aktivität durch UDP-GlcNAc um ca. 51% bzw. 80%). Es gibt Hinweise darauf, dass die Inhibition eines Proteins mit der Aktivität einer GFAT-1 in tierischen Organismen darauf zurückzuführen ist, dass bei erhöhten UDP-GlcNAc Konzentrationen eine O-Glucose-N-Acetylglucosamin Glycosylierung der betreffenden Proteine erfolgt. Ob eine Regulation der Aktivität von Proteinen mittels O-Glycosylierung auch in pflanzlichen Zellen vorkommt, ist zurzeit nicht eindeutig geklärt (Huber und Hardin, 2004, Current Opinion in Plant Biotechnology 7, 318-322).
Proteine mit der Aktivität einer bakteriellen GFAT zeichnen sich dadurch aus, dass sie nicht durch UDP-GlcNAc inhibiert werden (Kornfeld, 1967, J. Biol. Chem. 242(13), 3135-3141).
Proteine mit der Aktivität einer GFAT-1, Proteine mit der Aktivität einer GFAT-2, und auch Proteine mit der Aktivität einer bakteriellen GFAT werden durch das aus ihrer Reaktion entstehende Produkt Glucosamin-6-Phosphat inhibiert (Broschat et al., 2002, J. Biol. Cehm. 277(17), 14764-14770; Deng et al., 2005, Metabolic Engeneering 7, 201-214).

Unter dem Begriff "Protein mit der Aktivität einer Glutamin:Fructose-6-Phosphat Amidotransferase der isoform I (GFAT-1)" soll im Zusammenhang mit der vorliegenden Erfindung ein Protein verstanden werden, das die Aktivität einer GFAT aufweist und dessen Aktivität durch Phosphorylierung mittels einer cAMP abhängigen Proteinkinase inhibiert wird.

Unter dem Begriff "Protein mit der Aktivität einer Glutamin:Fructose-6-Phosphat Amidotransferase der Isoform II (GFAT-2)" soll im Zusammenhang mit der vorliegenden Erfindung ein Protein verstanden werden, das die Aktivität einer GFAT aufweist und das durch Phosphorylierung mittels einer cAMP abhängigen Proteinkinase aktiviert wird.

Unter dem Begriff "Protein mit der Aktivität einer bakteriellen Glutamin:Fructose-6-Phosphat Amidotransferase (bakterielle GFAT)" soll im Zusammenhang mit der vorliegenden Erfindung ein Protein verstanden werden, das die Aktivität einer GFAT aufweist und dessen Aktivität nicht durch UDP-GlcNAc inhibiert wird. Alternativ können "Proteine mit der Aktivität einer bakteriellen GFAT" auch als "Proteine mit der Aktivität einer nicht eukaryontischen GFAT" bezeichnet werden.

Erfindungsgemäß kann das fremde Nucleinsäuremolekül codierend ein Protein mit der enzymatischen Aktivität einer GFAT aus einem beliebigen Organismus stammen, bevorzugt stammt besagtes Nucleinsäuremolekül aus Bakterien, Pilzen, Tieren, Pflanzen oder Viren, besonders bevorzugt aus Säugetieren, Pflanzen oder Bakterien und insbesondere bevorzugt aus Maus oder *Escherichia coli.*
Bezüglich Viren stammt das das fremde Nucleinsäuremolekül codierend ein Protein mit der enzymatischen Aktivität einer GFAT vorzugsweise aus einem Virus, der Algen infiziert, bevorzugt aus einem Virus, der Algen der Gattung *Chlorella* infiziert, besonders bevorzugt aus einem *Paramecium bursaria Chlorella* Virus und insbesondere bevorzugt aus einem *Paramecium bursaria Chlorella* Virus eines H1 Stammes.
Anstelle eines natürlich vorkommenden Nucleinsäuremoleküls, codierend ein Protein mit der enzymatischen Aktivität einer GFAT kann auch ein mittels Mutagenese erzeugtes Nucleinsäuremolekül in Pflanzenzellen eingeführt werden, wobei sich besagtes mutagenisierte fremde Nucleinsäuremolekül dadurch auszeichnet, dass es ein Protein mit der enzymatischen Aktivität einer GFAT codiert, das eine verringerte Inhibition durch Stoffwechselmetabolite (z.B. des Glucosaminstoffwechsels) aufweist. Die Herstellung solcher mutagenisierten Nucleinsäuremoleküle ist für ein Protein mit der enzymatischen Aktivität einer GFAT aus *Escherichia coli* exemplarisch beschrieben in Deng et al. (2005, Metabolic Engineering 7, 201-214; WO 04 003175). Mutanten für ein Protein mit der Aktivität einer GFAT aus Maus sind z.B. beschrieben bei Hu et al. (2004, J. Biol. Chem. 279 (29), 29988-29993).

Nucleinsäuremoleküle, die ein Protein mit der Aktivität einer GFAT codieren sind dem Fachmann bekannt und in der Literatur beschrieben. So sind Nucleinsäuremoleküle, die ein Protein mit der Aktivität einer GFAT codieren aus Viren z.B. für den *Chlorella* Virus k2 (EMBL acc No AB107976.1), aus Bakterien z.B. für *Escherichia coli* (Dutka-Malen, 1988, Biochemie 70 (2), 287-290; EMBL acc No: L10328.1), aus Pilzen für z.B. *Saccharomyces cerevisiae* (EMBL acc No AF334737.1, Watzele et al., 1989, J. Biol. Chem. 264, 8753-8758), *Aspergillus niger* (EMBL acc No AY594332.1), *Candida albicans* (EMBL acc No X94753.1), aus Insekten für z.B. *Aedes aegyti* (Kato et al., 2002, Insect. Biol. 11 (3), 207,216; EMBL acc No AF399922.1), *Drosophila melanogaster* (GFAT-1: EMBL acc No Y18627.1, GFAT-2: NCBI acc No NM_143360.2), aus Algen für *Volvariella volvacea* (EMBL acc No AY661466.1), aus Vertebraten für z.B. *Homo sapiens* (GFAT-1: EMBL acc No AF334737.1; GFAT-2: NCBI acc No BC000012.2, Oki et al., 1999, Genomics 57 (2), 227-34), *Mus musculus* (GFAT-1: EMBL acc No AF334736.1; GFAT-2: EMBL acc No AB016780.1), oder aus Pflanzen für z.B. *Arabidopsis thaliana* (EMBLI acc No AP001297.1; cds NCBI acc No BAB03027.1) beschrieben.

In einer bevorzugten Ausführungsform wird in erfindungsgemäßen Verfahren zur Herstellung modifizierter Stärke ein fremdes Nucleinsäuremolekül codierend ein Protein mit der Aktivität einer GFAT-2 oder einer bakteriellen GFAT in eine Pflanzenzelle eingeführt.

In einer bevorzugten Ausführungsform betrifft die vorliegende Erfindung erfindungsgemäße Verfahren zur Herstellung modifizierter Stärke, worin das fremde Nucleinsäuremolekül codierend ein Protein mit der Aktivität einer GFAT ausgewählt ist, aus der Gruppe bestehend aus

a) Nucleinsäuremolekülen, die ein Protein mit der unter SEQ ID NO 2 oder Protein mit der unter SEQ ID NO 5 angegebenen Aminosäuresequenz codieren;

b) Nucleinsäuremolekülen, die ein Protein codieren, dessen Sequenz eine Identität von mindestens 60%, vorzugsweise von mindestens 70%, bevorzugt von mindestens 80%, besonders bevorzugt von mindestens 90%, insbesondere bevorzugt von mindestens 95% und im speziellen bevorzugt von mindestens 97% zu der unter SEQ ID NO 2 unter SEQ ID NO 5 angegebenen Aminosäuresequenz aufweisen;

c) Nucleinsäuremolekülen, die die unter SEQ ID NO 1 oder die unter SEQ ID NO 3 oder die unter SEQ ID NO 4 dargestellte Nucleotidsequenz oder eine komplementäre Sequenz umfassen;

d) Nucleinsäuremolekülen, welche zu den unter a) oder c) beschriebenen Nucleinsäuresequenzen eine Identität von mindestens 70%, vorzugsweise von mindestens 80%, bevorzugt von mindestens 90%, besonders bevorzugt von mindestens 95%, insbesondere bevorzugt von mindestens 97% und im speziellen bevorzugt von mindestens 98% aufweisen;

e) Nucleinsäuremolekülen, welche mit mindestens einem Strang der unter a) oder c) beschriebenen Nucleinsäuresequenzen unter stringenten Bedingungen hybridisieren;

f) Nucleinsäuremolekülen, deren Nucleotidsequenz aufgrund der Degeneration des genetisches Codes von der Sequenz der unter a) oder c) genannten Nucleinsäuremoleküle abweicht; und

g) Nucleinsäuremolekülen, die Fragmente, allelische Varianten und/oder Derivate der unter a), b), c), d), e) oder f) genannten Nucleinsäuremoleküle darstellen.

Der Begriff "Hybridisierung" bedeutet im Rahmen der vorliegenden Erfindung eine Hybridisierung unter konventionellen Hybridisierungsbedingungen, vorzugsweise unter stringenten Bedingungen, wie sie beispielsweise in Sambrock et al., Molecular Cloning, A Laboratory Manual, 2. Aufl. (1989) Cold Spring Harbor Laboratory Press, Cold Spring Harbor, NY) beschrieben sind. Besonders bevorzugt bedeutet "Hybridisierung" eine Hybridisierung unter den folgenden Bedingungen:

Hybridisierungspuffer:
2xSSC; 10xDenhardt-Lösung (Fikoll 400+PEG+BSA; Verhältnis 1:1:1); 0,1% SDS; 5 mM EDTA; 50 mM Na2HPO4; 250 μg/ml Heringssperma DNA; 50 μg/ml tRNA; oder 25 M Natriumphoshphatpuffer pH 7,2; 1 mM EDTA; 7% SDS
Hybridisierungstemperatur: T=65 bis 68°C
Waschpuffer: 0,1xSSC; 0, 1 % SDS
Waschtemperatur: T=55 bis 65°C
Hybridisierungsdauer: Über Nacht (ca. 12 bis 16 Stunden)
Waschdauer: 15 Minuten

Nucleinsäuremoleküle, die mit den genannten Molekülen hybridisieren können z.B. aus genomischen oder aus cDNA-Bibliotheken isoliert werden. Die Identifizierung und Isolierung derartiger Nucleinsäuremoleküle kann dabei unter Verwendung der genannten Nucleinsäuremoleküle oder Teile dieser Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY) oder durch Amplifikation mittels PCR.

Als Hybridisierungsprobe zur Isolierung einer Nucleinsäuresequenz, codierend ein Protein mit der Aktivität einer GFAT können z.B. Nucleinsäuremoleküle verwendet werden, die exakt die oder im Wesentlichen die unter SEQ ID NO 1 oder zu der unter SEQ ID NO 3 oder zu der unter SEQ ID NO 4 angegebene Nucleotidsequenz oder Teile dieser Sequenzen aufweisen.

Bei den als Hybridisierungsprobe verwendeten Fragmenten kann es sich auch um synthetische Fragmente oder Oligonucleotide handeln, die mit Hilfe der gängigen Synthesetechniken hergestellt wurden und deren Sequenz im wesentlichen mit der eines im Rahmen der vorliegenden Erfindung beschriebenen Nucleinsäuremoleküls übereinstimmt. Hat man Gene identifiziert und isoliert, die mit den im Rahmen der vorliegenden Erfindung beschriebenen Nucleinsäuresequenzen hybridisieren, sollte eine Bestimmung der Sequenz und eine Analyse der Eigenschaften der von dieser Sequenz codierten Proteine erfolgen, um festzustellen, ob es sich um Proteine handelt, die die Aktivität einer GFAT aufweisen. Methoden, wie bestimmt werden kann, ob ein Protein die Aktivität eines Proteins mit der Aktivität einer GFAT aufweist, sind dem Fachmann bekannt und u.a. in der angegebenen Literatur beschrieben (z.B. Mayer et al., 1968, Plant Physiol. 43, 1097-1107; Deng et al., 2005, Metabolic Engeneering 7, 201-214), GFAT-1 oder GFAT-2 (z.B. Hu et al., 2004, J. Biol.

Chem. 279 (29), 29988-29993).

Die mit den im Rahmen der vorliegenden Erfindung beschriebenen Nucleinsäuremolekülen hybridisierenden Moleküle umfassen insbesondere Fragmente, Derivate und allelische Varianten der genannten Nucleinsäuremoleküle. Der Begriff "Derivat" bedeutet im Zusammenhang mit der vorliegenden Erfindung, dass die Sequenzen dieser Moleküle sich von den Sequenzen der oben beschriebenen Nucleinsäuremoleküle an einer oder mehreren Positionen unterscheiden und einen hohen Grad an Identität zu diesen Sequenzen aufweisen. Die Abweichungen zu den oben beschriebenen Nucleinsäuremolekülen können dabei z.B. durch Deletion, Addition, Substitution, Insertion oder Rekombination entstanden sein.

Der Begriff "Identität" bedeutet im Zusammenhang mit der vorliegenden Erfindung eine Sequenzidentität über die gesamte Länge der codierenden Region eines Nucleinsäuremoleküls oder die gesamte Länge einer Aminosäuresequenz, die ein Protein codiert, von vorzugsweise mindestens 60%, insbesondere eine Identität von mindestens 70%, bevorzugt von mindestens 80%, besonders bevorzugt von mindestens 90%, insbesondere bevorzugt von mindestens 95% und im speziellen bevorzugt von mindestens 98%. Unter dem Begriff "Identität" soll im Zusammenhang mit der vorliegenden Erfindung die Anzahl der übereinstimmenden Aminosäuren/Nucleotide (Identität) mit anderen Proteinen/Nucleinsäuren, ausgedrückt in Prozent verstanden werden. Bevorzugt wird die Identität betreffend ein Protein mit der Aktivität einer GFAT durch Vergleiche der unter SEQ ID NO 2 oder SEQ ID NO 5 angegebenen Aminosäuresequenz bzw. die Identität betreffend ein Nucleinsäuremolekül codierend ein Protein mit der Aktivität einer GFAT durch Vergleiche der unter SEQ ID NO 1 oder SEQ ID NO 6 oder SEQ ID NO 3 oder SEQ ID NO 4 angegebenen Nucleinsäuresequenz zu anderen Proteinen/Nucleinsäuren mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., Nucleic Acids Research 22 (1994), 4673-4680) ermittelt. ClustalW wird öffentich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK), heruntergeladen werden.

Vorzugsweise wird das ClustalW Computerprogramm der Version 1.8 benutzt, um die Identität zwischen im Rahmen der vorliegenden Erfindung beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Vorzugsweise wird das ClustalW Computerprogramm der Version 1.8 benutzt, um die Identität zwischen z.B. der Nucleotidsequenz der im Rahmen der vorliegenden Erfindung beschriebenen Nucleinsäuremoleküle und der Nucleotidsequenz von anderen Nucleinsäuremolekülen zu bestimmen. Dabei sind folgende Parameter einzustellen:

KTUPLE=2, TOPDIAGS=4, PAIRGAP=5, DNAMATRIX:IUB, GAPOPEN=10, GAPEXT=5, MAXDIV=40, TRANSITIONS: unweighted.

Identität bedeutet ferner, dass funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Spezies oder um Mutationen handeln, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten. Eine spezielle Form von Derivaten stellen z.B. Nucleinsäuremoleküle dar, die auf Grund der Degeneration des genetischen Codes von im Rahmen der vorliegenden Erfindung beschriebenen Nucleinsäuremolekülen abweichen.

In einer weiteren bevorzugten Ausführungsform betrifft die vorliegende Erfindung erfindungsgemäße Verfahren zur Herstellung modifizierter Stärke worin das fremde Nucleinsäuremolekül codierend ein Protein mit der enzymatischen Aktivität einer GFAT dadurch gekennzeichnet ist, dass die Codons besagten Nucleinsäuremoleküls verändert sind, im Vergleich zu den Codons des Nucleinsäuremoleküls, welche besagtes Protein mit der enzymatischen Aktivität einer GFAT des Ursprungsorganismus codieren. Besonders bevorzugt sind die Codons des fremden Nucleinsäuremoleküls codierend ein Protein mit der enzymatischen Aktivität einer GFAT dahingehend verändert, dass sie an die Häufigkeit der Verwendung der Codons der Pflanzenzelle oder der Pflanze, in dessen Genom sie integriert sind oder werden, angepasst sind.

Aminosäuren können auf Grund der Degeneration des genetischen Codes durch ein oder mehrere Codons codiert werden. Unterschiedliche Organismen verwenden die jeweils für eine Aminosäure codierenden Codons mit unterschiedlicher Häufigkeit. Das Anpassen der Codons einer codierenden Nucleinsäuresequenz an die Häufigkeit ihrer Verwendung in der Pflanzenzelle oder in der Pflanze, in dessen Genom die zu exprimierende Sequenz integriert werden soll, kann zu einer erhöhten Menge an translatiertem Protein und/oder zur Stabilität der betreffenden mRNA in den betreffenden Pflanzenzellen oder Pflanzen beitragen. Der Fachmann kann die Häufigkeit der Verwendung von Codons in betreffenden Pflanzenzellen oder Pflanzen ermitteln, indem er möglichst viele codierende Nucleinsäuresequenzen des betreffenden Organismus dahingehend untersucht, wie häufig bestimmte Codons für die Codierung einer bestimmten Aminosäure verwendet werden. Die Häufigkeit der Verwendung von Codons bestimmter Organismen ist dem Fachmann geläufig und kann mit Hilfe von Computerprogrammen einfach und schnell durchgeführt werden. Entsprechende Computerprogramme sind öffentlich zugänglich und werden u.a. im Internet frei zur Verfügung gestellt (z.B. http://gcua.schoedl.de/; http://www.kazusa.or.jp/codon/; http://www.entelechon.com/eng/cutanalysis.html). Das Anpassen der Codons einer codierenden Nucleinsäuresequenz an die Häufigkeit ihrer Verwendung in der Pflanzenzelle oder in der Pflanze, in dessen Genom die zu exprimierende Sequenz eingeführt werden soll, kann durch *in vitro* Mutagenese oder bevorzugt durch Neusynthese der Gensequenz erfolgen. Methoden zur Neusynthese von Nucleinsäuresequenzen sind dem Fachmann bekannt. Eine Neusynthese kann z.B. dadurch erfolgen, dass zunächst einzelne Nucleinsäureoligonucleotide synthetisiert werden, diese mit zu ihnen komplementären Oligonucleotiden hybridisiert werden, so dass sie einen DNA-Doppelstrang ausbilden, bevor die einzelnen doppelsträngigen Oligonucleotide so miteinander ligiert werden, dass die gewünschte Nucleinsäuresequenz erhalten wird. Die Neusynthese von Nucleinsäuresequenzen inklusive der Anpassung der Häufigkeit der Verwendung der Codons an einen bestimmten Zielorganismus kann auch als Auftrag an Firmen, die dieses als Dienstleistung anbieten, vergeben werden (z.B. Entelechon GmbH, Regensburg, Germany).

Für die Einführung von Nucleinsäuremolekülen in eine pflanzliche Wirtszelle steht eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten (Übersicht in "Transgenic Plants", Leandro ed., Humana Press 2004, ISBN 1-59259-827-7).
Die Verwendung der Agrobakterien-vermittelten Transformation von Pflanzenzellen ist intensiv untersucht und ausreichend in EP 120516; Hoekema (1985, IN: The Binary Plant Vector System Offsetdrukkerij Kanters B.V. Alblasserdam, Chapter V); Fraley et al., (1985, Crit. Rev. Plant Sci. 4, 1-46) und bei An et al. (1985, EMBO J. 4, 277-287) beschrieben worden. Für die Transformation von Kartoffel, siehe z.B. Rocha-Sosa et al. (1989, EMBO J. 8, 29-33), für die Transformation von Tomatenpflanzen z.B. US 5,565,347.

Auch die Transformation monokotyler Pflanzen mittels auf *Agrobacterium* Transformation basierender Vektoren wurde beschrieben (Chan et al., 1993, Plant Mol. Biol. 22, 491-506; Hiei et al., 1994, Plant J. 6, 271-282; Deng et al, 1990, Science in China 33, 28-34; Wilmink et al., 1992, Plant Cell Reports 11, 76-80; May et al., 1995, Bio/Technology 13, 486-492; Conner und Domisse, 1992, Int. J. Plant Sci. 153, 550-555; Ritchie et al, 1993, Transgenic Res. 2, 252-265).
Ein alternatives System zur Transformation von monokotylen Pflanzen ist die Transformation mittels des biolistischen Ansatzes (Wan und Lemaux, 1994, Plant Physiol. 104, 37-48; Vasil et al., 1993, Bio/Technology 11, 1553-1558; Ritala et al., 1994, Plant Mol. Biol. 24, 317-325; Spencer et al., 1990, Theor. Appl. Genet. 79, 625-631), die Protoplastentransformation, die Elektroporation von partiell permeabilisierten Zellen, die Einbringung von DNA mittels Glasfasern. Insbesondere die Transformation von Mais wird in der Literatur mehrfach beschrieben (vgl. z. B. WO95/06128, EP0513849, EP0465875, EP0292435; Fromm et al., 1990, Biotechnology 8, 833-844; Gordon-Kamm et al., 1990, Plant Cell 2, 603-618; Koziel et al., 1993, Biotechnology 11, 194-200; Moroc et al., 1990, Theor. Appl. Genet. 80, 721-726). Die Transformation von anderen Gräsern, wie z.B. der Rutenhirse (switchgrass, *Panicum virgatum*) ist ebenfalls beschrieben (Richards et al., 2001, Plant Cell Reporters 20, 48-54).
Auch die erfolgreiche Transformation anderer Getreidearten wurde bereits beschrieben, z.B. für Gerste (Wan und Lemaux, s.o.; Ritala et al., s.o.; Krens et al., 1982, Nature 296, 72-74) und für Weizen (Nehra et al., 1994, Plant J. 5, 285-297; Becker et al., 1994, Plant Journal 5, 299-307). Alle vorstehenden Methoden sind im Rahmen der vorliegenden Erfindung geeignet.

Pflanzen, in die ein fremdes Nucleinsäuremolekül codierend ein Protein mit der Aktivität einer GFAT eingeführt wurde, lassen sich im Vergleich zu entsprechenden nicht genetisch modifizierten Wildtyp-Pflanzen unter anderem dadurch unterscheiden, dass sie ein fremdes Nucleinsäuremolekül enthalten, das natürlicherweise in Wildtyp-Pflanzen nicht vorkommt oder dadurch, dass ein solches Molekül an einem Ort im Genom der Pflanze integriert vorliegt, an dem es bei Wildtyp-Pflanzen nicht vorkommt, d.h. in einer anderen genomischen Umgebung. Ferner lassen sich derartige Pflanzen von nicht genetisch modifizierten modifizierten Wildtyp-Pflanzen dadurch unterscheiden, dass sie mindestens eine Kopie des fremden Nucleinsäuremoleküls stabil integriert in ihr Genom enthalten, gegebenenfalls zusätzlich zu natürlicherweise in den Wildtyp-Pflanzen vorkommenden Kopien eines solchen Moleküls. Handelt es sich bei dem in die genetisch modifizierten Pflanzen eingeführten fremden Nucleinsäuremolekül um zusätzliche Kopien zu bereits natürlicherweise in den Wildtyp-Pflanzen vorkommenden Molekülen, so lassen sich die genetisch modifizierten Pflanzen von Wildtyp-Pflanzen insbesondere dadurch unterscheiden, dass diese zusätzliche Kopie an Orten im Genom lokalisiert ist, an denen sie bei Wildtyp-Pflanzen nicht vorkommt.

Nachgewiesen werden kann ein in eine Pflanzenzelle eingeführtes fremdes Nucleinsäuremolekül durch genetische und/oder molekularbiologische Methoden.

Ein in eine Pflanzenzelle eingeführtes und Nucleinsäuremolekül kann u.a. mit Hilfe der Southern-Blot Analyse der RFLP-Analyse (Restriction Fragment Length Polymorphism) (Nam et al., 1989, The Plant Cell 1, 699-705; Leister and Dean, 1993, The Plant Journal 4 (4), 745-750), auf PCR basierenden Methoden, wie z.B. die Analyse von amplifizierten Fragment Längenunterschieden (Amplified Fragment Length Polymorphism, AFLP) (Castiglioni et al., 1998, Genetics 149, 2039-2056; Meksem et al., 2001, Molecular Genetics and Genomics 265, 207-214; Meyer et al., 1998, Molecular and General Genetics 259, 150-160) oder der Verwendung von mit Restriktionsendonucleasen geschnittenen amplifizierten Fragmenten (Cleaved Amplified Polymorphic Sequences, CAPS) (Konieczny und Ausubel, 1993, The Plant Journal 4, 403-410; Jarvis et al., 1994, Plant Molecular Biology 24, 685-687; Bachem et al., 1996, The Plant Journal 9 (5), 745-753) nachgewiesen werden.

Die Regeneration der Pflanzen gemäß Schritt b) der erfindungsgemäßen Verfahren zur Herstellung modifizierter Stärke kann nach dem Fachmann bekannten Methoden erfolgen (z.B. beschrieben in "Plant Cell Culture Protocols", 1999, edt. by R.D. Hall, Humana Press, ISBN 0-89603-549-2).

Die Erzeugung weiterer Pflanzen der erfindungsgemäßen Verfahren zur Herstellung modifizierter Stärke kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt. Die Auswahl erfolgt dabei bevorzugt in der Weise, dass die weiteren Pflanzen die in den vorangegangenen Schritten eingeführten fremden Nucleinsäuremoleküle aufweisen.

Bei den in erfindungsgemäßen Verfahren zur Herstellung modifizierter Stärke verwendeten Pflanzenzellen, in die ein fremdes Nucleinsäuremolekül eingeführt wird, kann es sich um Pflanzenzellen von Pflanzen einer beliebigen Pflanzenspezies handeln, d.h. sowohl um monokotyle als auch dikotyle Pflanzen. Vorzugsweise handelt es sich um Nutzpflanzen, d.h. Pflanzen, die vom Menschen kultiviert werden für Zwecke der Ernährung von Mensch und Tier (z.B. Mais, Reis, Weizen, Roggen, Hafer, Gerste, Maniok, Kartoffel, Tomate, Rutenhirse (*Panicum virgatum*), Sago, Mungbohne, Erbse, Sorghum, Möhre. Besonders bevorzugt handelt es sich um Reis-Mais-, Weizen- oder Kartoffelpflanzen.

Verfahren zur Extraktion der Stärke aus Pflanzen oder von Stärke speichernden Teilen von Pflanzen sind dem Fachmann bekannt. Weiterhin sind Verfahren zur Extraktion der Stärke aus verschiedenen Stärke speichernden Pflanzen beschrieben, z. B. in Starch: Chemistry and Technology (Hrsg.: Whistler, BeMiller und Paschall (1994), 2. Ausgabe, Academic Press Inc. London Ltd; ISBN 0-12-746270-8; siehe z.B. Kapitel XII, Seite 412-468: Mais und Sorghum Stärken: Herstellung; von Watson; Kapitel XIII, Seite 469-479: Tapioca, Arrowroot und Sago Stärken: Herstellung; von Corbishley und Miller; Kapitel XIV, Seite 479-490: Kartoffelstärke: Herstellung und Verwendungen; von Mitch; Kapitel XV, Seite 491 bis 506: Weizenstärke: Herstellung, Modifizierung und Verwendungen; von Knight und Oson; und Kapitel XVI, Seite 507 bis 528: Reisstärke: Herstellung und Verwendungen; von Rohmer und Klem; Maisstärke: Eckhoff et al., Cereal Chem. 73 (1996), 54-57, die Extraktion von Maisstärke im industriellen Maßstab wird in der Regel durch das so genannte "wet milling" erreicht.). Vorrichtungen, die für gewöhnlich bei Verfahren zur Extraktion von Stärke von Pflanzenmaterial verwendet werden, sind Separatoren, Dekanter, Hydrocyclone, Sprühtrockner und Wirbelschichttrockner.

In einer bevorzugten Ausführungsform handelt es sich bei dem erfindungsgemäßen Verfahren zur Herstellung einer modifizierten Stärke um ein Verfahren zur Herstellung einer resistenten Stärke (RS).

In einer weiteren bevorzugten Ausführungsform umfassen erfindungsgemäße Verfahren zur Herstellung einer modifizierten Stärke zusätzlich einen Verfahrensschritt, worin die Stärke nach dem Isolieren aus der Pflanze erhitz oder verkleistert (gelatinisiert) wird.

Eine bevorzugte Ausführungsform erfindungsgemäßer Verfahren zur Herstellung einer modifizierten Stärke betrifft Verfahren, worin die modifizierte Stärke nach Erhitzen oder Verkleisterung (Gelatinisieren) einen Anteil an resistenter Stärke (RS) von mindestens 40%, bevorzugt mindestens 45%, besonders bevorzugt mindestens 50%, insbesondere bevorzugt mindestens 55% und speziell bevorzugt mindestens 60% aufweist.

Eine weitere bevorzugte Ausführungsform erfindungsgemäßer Verfahren zur Herstellung einer modifizierten Stärke betrifft Verfahren, worin die modifizierte Stärke nach Erhitzen oder Verkleisterung (Gelatinisieren) einen Anteil an resistenter Stärke (RS) von 40% bis 80%, bevorzugt von 40% bis 70%, besonders bevorzugt von 40% bis 60% und insbesondere bevorzugt von 50% bis 60% aufweist.

Modifizierte Stärke, hergestellt mit einem erfindungsgemäßen Verfahren zur Herstellung einer modifizierten Stärke zeichnen sich vorzugsweise weiterhin dadurch aus, dass sie eine Stärke synthetisieren, die einen verringerten Anteil an schnell verdaulicher Stärke (RDS) aufweist.

Daher betrifft eine weitere bevorzugte Ausführungsform erfindungsgemäßer Verfahren zur Herstellung einer modifizierten Stärke Verfahren, worin die modifizierte Stärke nach Erhitzen oder Verkleisterung (Gelatinisieren) einen Anteil an schnell verdaulicher Stärke (RDS) von maximal 20%, bevorzugt maximal 17%, besonders bevorzugt maximal 16% und insbesondere bevorzugt maximal 15% aufweist.

Eine weitere bevorzugte Ausführungsform erfindungsgemäßer Verfahren zur Herstellung einer modifizierten Stärke betrifft Verfahren, worin die modifizierte Stärke nach Erhitzen oder Verkleisterung (Gelatinisieren) einen Anteil an schnell verdaulicher Stärke (RDS) von 10% bis 18%, bevorzugt von 12% bis 18%, besonders bevorzugt von 13% bis 17% und insbesondere bevorzugt von 15% bis 17% aufweist.

Modifizierte Stärke, hergestellt mit einem erfindungsgemäßen Verfahren zur Herstellung einer modifizierten Stärke zeichnet sich vorzugsweise auch dadurch aus, dass sie einen verringerten Anteil an langsam verdaulicher Stärke (SDS) aufweist.

Daher betrifft eine weitere bevorzugte Ausführungsform erfindungsgemäßer Verfahren zur Herstellung einer modifizierten Stärke Verfahren, worin die modifizierte Stärke nach Erhitzen oder Verkleisterung (Gelatinisieren) einen Anteil an langsam verdaulicher Stärke (SDS) von höchstens 45%, bevorzugt höchstens 40%, besonders bevorzugt höchstens 35%, insbesondere bevorzugt höchstens 30% und speziell bevorzugt höchstens 25% aufweist.

Eine weitere bevorzugte Ausführungsform erfindungsgemäßer Verfahren zur Herstellung einer modifizierten Stärke betrifft Verfahren, worin die modifizierte Stärke nach Erhitzen oder Verkleisterung (Gelatinisieren) einen Anteil an langsam verdaulicher Stärke (SDS) von 20% bis 45%, bevorzugt von 20% bis 40%, besonders bevorzugt von 25% bis 45% und insbesondere bevorzugt von 25% bis 42% aufweist.

Im Zusammenhang mit der vorliegenden Erfindung soll unter dem Begriff "resistente Stärke (RS)" der Anteil an Stärke verstanden werden, der nach nach Erhitzen oder Verkleisterung (Gelatinisieren) und anschließendem Verdau mit Pankreatin nach 120 Minuten nicht verdaut wurde.

Dem Fachmann ist bekannt, wie er eine gelatinisierte Stärke herstellt. Dazu wird Stärke unter rühren erhitzt, bis sich die Kornstruktur der Stärke auflöst. Das Gelatinisieren kann z.B. mit Hilfe von für die Stärkeanalytik konzipierten Geräten wie einem Rapid Visco Analyser (Newport Scientific) oder eines Brabenders (Brabender) erfolgen.

Eine bevorzugte Methode zum Gelatinisieren von Stärke ist unter allgemeine Methoden, Punkt 7 beschrieben.

Dem Fachmann ist ebenfalls bekannt, wie er den Anteil an resisitenter Stärke bestimmt. Der Anteil an resisitenter Stärke kann z.B. mit der AOAC Methode 2002.02 oder mit der AACC Methode 32-40 bestimmt werden.

Im Zusammenhang mit der vorliegenden Erfindung erfolgt die Bestimmung des RS-Anteils von Stärke vorzugsweise mittels der Methode von Englyst et al. (Europ. J. of Clinical Nutrition 46 (Suppl. 2), (1992), S 33-50, siehe insbesondere folgende Abschnitte aus Englyst et al., Seite S35-S36: "Reagents, Apparatus, Spectrophotometer"; Seite S36-S37, Absatz "Measurement of free glucose (FG)"; Seite S38, Absatz "Measurement of RDS and SDS"). Als resistenten Stärkeanteil (RS) der Stärke bezeichnet man den Anteil der eingewogenen Stärkeprobe (Trockengewicht), der in der beschriebenen Methode nach 120 Minuten nicht als Glucose freigesetzt wird. Er ergibt sich demnach gemäß folgender Formel:

$$\text{RS [\%]} = 100\% - 100\% \times (\text{freigesetzte Glucose nach 120 Minuten in mg}) / (\text{Trockengewicht Stärke in mg})$$

Im Zusammenhang mit der vorliegenden Erfindung soll unter dem Begriff "Anteil an schnell verdaulicher Stärke (RDS)" (= Rapidly Digestible Starch) der Anteil einer Stärke verstanden werden, der in der oben genannten Methode von Englyst et al. zur Bestimmung des RS-Anteils nach 20 Minuten als Glucose freigesetzt wird. Die Angabe in Gewichtsprozent bezieht sich hierbei auf das für die Bestimmung eingesetzte Trockengewicht der Stärkeprobe. Demnach gilt im Zusammenhang mit der vorliegenden Erfindung:

$$\text{RDS [\% ]} = 100\% \times (\text{freigesetzte Glucose nach 20 Minuten [mg]}) / (\text{Trockengewicht Stärke [mg]})$$

Im Zusammenhang mit der vorliegenden Erfindung soll unter "dem Anteil an langsam verdaulicher Stärke (SDS)" (= Slowly Digestible Starch) der Anteil einer Stärke verstanden werden, der in der oben genannten Methode von Englyst et al. zur Bestimmung des RS-Gehaltes in der Zeitspanne zwischen 20 Minuten und 120 Minuten als Glucose freigesetzt wird. Die Angabe in Gewichtsprozent bezieht sich hierbei auf das Trockengewicht der Stärkeprobe. Demnach gilt im Zusammenhang mit der vorliegenden Erfindung:

$$\text{SDS [\%]} = (\text{freigesetzte Glucose nach 120 Minuten [\%]}) - (\text{freigesetzte Glucose nach 20 Minuten [\%]})$$

Weiterhin zeichnet sich modifizierte Stärke, hergestellt nach einem erfindungsgemäßen Verfahren zur Herstellung einer modifizierten Stärke vorzugsweise dadurch aus, dass sie eine verringerte Korngröße aufweist im Vergleich zu Stärke, die aus entsprechenden Wildtyp-Pfdlanzen isoliert wurde.

Daher betrifft eine weitere bevorzugte Ausführungsform erfindungsgemäßer Verfahren zur Herstellung einer modifizierten Stärke Verfahren zur Herstellung einer modifizierten Stärke, die eine verringerte Korngröße aufweist im Vergleich zu Stärke, die aus entsprechenden Wildtyp-Pfdlanzen isoliert wurde. Bevorzugt beträgt die mittlere Korngröße der Stärke höchstens 60%, besonders bevorzugt höchstens 55%, insbesondere bevorzugt höchstens 50% im Vergleich zur Korngröße von Stärke, die aus entsprechenden Wildtyp-Pfdlanzen isoliert wurde.

Betreffend eine Stärke, isoliert aus einer Kartoffelpflanze oder aus Knollen einer Kartoffelpflanze beträgt die mittlere Korngröße der mittels eines erfindungsgemäßen Verfahrens zur Herstellung einer modifizierten Stärke hergestellten Stäke vorzugsweise 15 $\mu$m bis 30 $\mu$m, bevorzugt 17 $\mu$m bis 28 $\mu$m, besonders bevorzugt 19 $\mu$m bis 27 $\mu$m und insbesondere bevorzugt 20 $\mu$m bis 26 $\mu$m.

Methoden zur Bestimmung der Korngröße von Stärke sind dem Fachmann bekannt. Bevorzugt wird die Korngröße im Zusammenhang mit der vorliegenden Erfindung mittels der unter allgemeine Methoden, Punkt 6 e) beschriebenen Methode bestimmt.

Stärken mit verringerter Korngröße bieten gegenüber herkömmlichen Stärken den Vorteil, dass sie die Textur von Nahrungsmitteln, in die sie eingemischt werden, weniger stark verändern. Werden garnuläre Stärken zu Nahrungsmitteln hinzu gegeben, so hat dieses häufig negative Auswirkungen auf die Textur des Nahrungsmittels und beim Verzehr entsteht u.a. durch die Größe der Stärkekörner ein unerwünschtes, oft sandiges Mundgefühl ("mouth feel"). Dieser negative Effekt ist beim Vorliegen von kleineren Stärkekörnern weniger stark ausgeprägt. Daher eignen sich Stärken, hergestellt mit einem erfindungsgemäßen Verfahren zur Herstellung einer modifizierten Stärke besser für die Zumischung zu Nahrungsmitteln.

Weiterhin wurde überraschenderweise gefunden, dass Stärken, hergestellt mit einem erfindungsgemäßen Verfahren zur Herstellung einer modifizierten Stärke veränderte Viskositätseigenschaften aufweisen können im Vergleich zu Stärke, die aus entsprechenden Wildtyp-Pflanzen isoliert wurde.

Die Viskositätseigenschaften von Stärken, denen auch die maximale Viskosität und die Endviskositat zuzurechnen sind, werden häufig vom Fachmann mit unterschiedlichen Methoden bestimmt. Eine schnelle und effiziente Methode zur Analyse der Verkleisterungseigenschaften bietet die RVA Analyse.
Ein Protokoll zur Durchführung der RVA Analyse ist unter Allgemeine Methoden, Punkt 6 c) beschrieben.

Daher betrifft die vorliegende Erfindung vorzugsweise auch erfindungsgemäße Verfahren zur Herstellung einer modifizierten Stärke, die veränderte Viskositätseigenschaften aufweist im Vergleich zu Stärke, die aus entsprechenden Wildtyp-Pflanzen isoliert wurde. Bevorzugt weisen Stärken, hergestellt mit einem erfindungsgemäßen Verfahren zur Herstellung einer modifizierten Stärke in der RVA Analyse eine verringerte maximale Viskosität (RVA Max) und/oder eine erhöhte Endviskosität auf im Vergleich zu Stärke, isoliert aus entsprechenden Wildtyp-Pflanzen.
Die maximale Viskosität in der RVA Analyse von Stärke, hergestellt nach einem erfindungsgemäßen Verfahren zur Herstellung einer modifizierten Stärke ist vorzugsweise um mindestens 25%, besonders bevorzugt um mindestens 30% und insbesondere bevorzugt um mindestens 50% verringert im Vergleich zu Stärke, die aus entsprechenden Wildtyp-Pflanzen isoliert wurde.
Die Endviskosität in der RVA Analyse von Stärke, hergestellt nach einem erfindungsgemäßen Verfahren zur Herstellung einer modifizierten Stärke ist vorzugsweise um mindestens 20%, besonders bevorzugt um mindestens 30% und insbesondere bevorzugt um mindestens 39% erhöht im Vergleich zu Stärke, die aus entsprechenden Wildtyp-Pflanzen isoliert wurde.

Der Begriff "Wildtyp-Pflanze" bedeutet im Zusammenhang mit der vorliegenden Erfindung, dass es sich um Pflanzen handelt, deren Zellen als Ausgangsmaterial für die Einführung eines fremden Nucleinsäuremoleküls codierend ein Protein mit der Aktivität einer Glutamin:Fructose-6-Phosphat Amidotransferase (GFAT) in einem erfindungsgemäßen Verfahren verwendet wurden, d.h. deren genetische Information, abgesehen von dem Vorliegen besagten fremden Nucleinsäuremoleküls, der einer mittels eines erfindungsgemäßen Verfahrens hergestellten Pflanze entspricht.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten wurden. Im Zusammenhang mit der vorliegenden Erfindung bedeutet der Begriff "entsprechend" im Zusammenhang mit Wildtyp-Pflanze, dass die Pflanzen, die miteinander verglichen werden, unter gleichen Kulturbedingungen aufgezogen wurden und dass sie ein gleiches (Kultur-) Alter aufweisen.

Stärken bilden nach Verkleisterung und anschließender Abkühlung häufig stabile Gele aus. Um Stärkegele herzustellen muss die kristalline Struktur von nativer Stärke zunächst durch Erhitzen in wässriger Suspension unter ständigem Rühren zerstört werden. Dieses kann mit dem Fachmann bekannten Methoden z.B. mit Hilfe eines Rapid Visco Analyser (Newport Scientific Pty Ltd., Investmet Support Group, Warriewod NSW 2102, Australien) durchgeführt werden. Zur Bestimmung der Gelfestigkeit werden die verkleisterten Stärkesuspensionen für eine gewisse Zeit gelagert und die durch Lagerung ausgebildeten Gele anschließend auf ihre Festigkeit hin untersucht. Die Gelfestigkeit kann mit dem Fachmann bekannten Methoden, z.B. mittels Verwendung eines so genannten "Texture Analysers" erfolgen. Eine im Zusammenhang mit der vorliegenden Erfindung bevorzugt zu verwendende Methode zur Bestimmung der Gelfestigkeit ist unter Allgemeine Methoden, Punkt 6 d) beschrieben.

Die Eigenschaft der Ausbildung von stabilen Gelen von Stärke wird bei der Herstellung von Nahrungsmitteln vielfältig genutzt.

Stärken, hergestellt nach einem erfindungsgemäßen Verfahren zeichnen sich vorzugsweise auch dadurch aus, dass sie nach Verkleisterung Gele mit erhöhter Festigkeit ausbilden.

Daher betrifft die vorliegende Erfindung vorzugsweise auch erfindungsgemäße Verfahren zur Herstellung einer modifizierten Stärke, die nach Verkleisterung Gele ausbildet, die eine erhöhte Festigkeit aufweisen (Gelfestigkeit) im Vergleich zu Stärke, die aus entsprechenden Wildtyp-Pflanzen isoliert wurde. Die Gelfestigkeit von Stärke, hergestellt nach einem erfindungsgemäßen Verfahren zur Herstellung einer modifizierten Stärke ist vorzugsweise um mindestens 100%, besonders bevorzugt um mindestens 200%, insbesondere bevorzugt um mindestens 250% im speziellen bevorzugt um mindestens 300% erhöht im Vergleich zu Stärke, die aus entsprechenden Wildtyp-Pflanzen isoliert wurde.

Stärken, hergestellt nach einem erfindungsgemäßen Verfahren zur Herstellung einer modifizierten Stärke, die eine erhöhte Endviskosität in der RVA Analyse aufweisen und die Gele mit erhöhter Festigkeit ausbilden, sind besonders geeignet für alle Anwendungen, bei welchen die Dickungsleistung, die Geliereigenschaften oder die Bindungseigenschaften zugesetzter Substanzen von Bedeutung sind. Um z.B. eine bestimmte Dickungsleistung oder die Ausbildung eines Gels in Nahrungsmitteln zu erlangen, muss von Stärke, hergestellt mit einem erfindungsgemäßen Verfahren zur Herstellung einer modifizierten Stärke weniger eingesetzt werden im Vergleich zu Stärke, isoliert aus Wildtyp-Pflanzen. Der Kaloriengehalt des betreffenden Nahrungsmittels kann dadurch zusätzlich zu dem durch den erhöhten RS Gehalt reduzierten Anteil weiter verringert werden, was für den Konsumenten die bereits beschriebenen Vorteile aufweist. Daher eignet sich die erfindungsgemäße Stärke besonders zur Herstellung von Nahrungsmitteln wie z.B. Backwaren, Fertignahrungsmitteln (Instant Food), Pudding, Suppen, Konfeket, Schokolade, Eiskrem, Panaden für Fisch- oder Fleisch, gefrorene Dessets oder extrudierte Snacks.

Mittels DSC Analyse kann der Übergang von Stärke aus der kristallinen Form in die amorphe Form dargestellt werden. Die mittels DSC ermittelten endothermen Kurvenverläufe sind durch verschiedene Parameter ($T_O$, $T_P$, $T_e$ und dH) näher charakterisiert. Die onset-Temperatur $T_O$ kennzeichnet den Beginn der thermischen Umwandlung. Am Wert für $T_P$ ($T_P$ = DSC Peaktemperatur) ist die Temperatur ablesbar, bei der die maximale thermische Umsetzung des kristallinen Materials erfolgt, während $T_e$ die Temperatur darstellt, bei der der Umwandlungsprozeß abgeschlossen ist (Endtemperatur).

Die DSC Umwandlungsenthalpie (deltaH) wird durch Berechnung der Kurvenfläche ermittelt. Sie stellt die Gesamtenergie dar, die für die Transformation notwendig ist.

Bevorzugt weisen Stärken, hergestellt nach einem erfindungsgemäßen Verfahren zur Herstellung einer modifizierten Stärke auch Veränderungen in der thermischen Stabilität auf im Vergleich zu Stärken, isoliert aus entsprechenden Wildtyp-Pflanzen.

Die thermische Stabilität von Stärken kann mit Hilfe der so genannten DSC ("Differential Scanning Calorimetrie") ermittelt werden. Die Methode der DSC ist dem Fachmann bekannt. Ergebnisse von DSC-Messungen werden u.a. zur Charakterisierung der thermischen Stabilität von RS genutzt. Die im Zusammenhang mit der vorliegenden Erfindung vorzugsweise zu verwendende DSC-Methode ist unter Allgemeine Methoden, Punkt 6 f) beschrieben..

In einer weiteren vorzugsweisen Ausführungsform betrifft die vorliegende Erfindung erfindungsgemäße Verfahren zur Herstellung einer modifizierten Stärke, worin die thermische Stabilität der Stärke eine erhöhte DSC onset-Temperatur ($T_O$) und/oder eine erhöhte DSC Peaktemperatur ($T_P$) aufweist im Vergleich zu Stärke, isoliert aus Wildtyp-Pflanzen.

Vorzugsweise weisen Stärken, hergestellt nach einem erfindungsgemäßen Verfahren zur Herstellung einer modifizierten Stärke eine um mindestes 1°C besonders bevorzugt um mindestens 2°C und insbesondere um mindestens 2,5°C erhöhte DSC onset-Temperatur ($T_O$) auf im Vergleich zu Stärke, isoliert aus entsprechenden Wildtyp-Pflanzen.

Vorzugsweise weisen Stärken, hergestellt nach einem erfindungsgemäßen Verfahren zur Herstellung einer Stärke eine um mindestes 1 °C besonders bevorzugt um mindestens 2°C und insbesondere um mindestens 3,5°C erhöhte DSC Peaktemperatur ($T_p$) auf im Vergleich zu Stärke, isoliert aus entsprechenden Wildtyp-Pflanzen.

Nach dem Erhitzen oder Verkleistern und anschließendem Abkühlen von Stärke bilden sich zumindest teilweise wieder je nach Art der Stärke mehr oder weniger kristalline Strukturen aus (Retrogradation). Der Anteil an kristallinen Strukturen in retrogradierter Stärke kann ebenfalls mit Hilfe der DSC Analyse durch Ermittlung der für den Übergang aus der kristallinen in die amorphe Form benötigten DSC Umwandlungsenergie (deltaH) bestimmt werden. Eine im Zusammenhang mit der vorliegenden Erfindung bevorzugte Methode zur Bestimmung des deltaH ist unter Allgemeine Methgoden, Punkt 6 f) beschrieben.

In einer weiteren vorzugsweisen Ausführungsform betrifft die vorliegende Erfindung erfindungsgemäße Verfahren zur Herstellung einer modifizierten Stärke, worin die thermische Stabilität von retrogradierter Stärke, hergestellt nach einem erfindungsgemäßen Verfahren zur Herstellung einer modifizierten Stärke eine erhöhte DSC Umwandlungsenthalpie (deltaH) aufweist im Vergleich zu Stärke, isoliert aus Wildtyp-Pflanzen.

Bevorzugt weisen Stärken, hergestellt nach einem erfindungsgemäßen Verfahren zur Herstellung einer Stärke nach Retrogradadtion eine um mindestes 1 J/g besonders bevorzugt um mindestens 1,5 J/g, insbesondere bevorzugt um mindestens 2,0 J/g und im speziellen bevorzugt von mindestens 2,5 J/g erhöhte DSC Umwandlungsenthalpie (deltaH) auf im Vergleich zu Stärke, die aus entsprechenden Wildtyp-Pflanzen isoliert und anschließend retrogradiert wurde.

Eine signifikante Veränderung des Amylosegehaltes oder des Gehaltes an in C-6-Position der Glucosemoleküle der Stärke gebundenem Phosphat in Stärke, hergestellt nach einem erfindungsgemäßen Verfahrens zur Herstellung einer modifizierten Stäke im Vergleich zu Stärke, isoliert aus entsprechenden Wildtyp-Pflanzen konnte nicht nachgewiesen werden.

Stärke erhältlich nach einem erfindungsgemäßen Verfahren zur Herstellung einer modifizierten Stärke, ist ebenfalls Gegenstand der vorliegenden Erfindung.

Die Verwendung eines Nucleinsäuremoleküls codierend ein Protein mit der Aktivität einer Glutamin:Fructose-6-Phosphat Amidotransferase (GFAT) zur Herstellung einer modifizierten Stärke ist ein weiterer Gegenstand der vorliegenden Erfindung.

Nahungsmittel (Lebensmittel), die eine erfindungsgemäße Stärke oder eine Stärke, hergestellt nach einem erfindungsgemäßen Verfahren zur Herstellung einer modifizierten Stärke aufweisen zeichnen sich dadurch aus, dass sie einen reduzierten Kaloriengehalt aufweisen. Weiterhin führt der hohe Gehalt an RS von Stärken, hergestellt mit einem erfindungsgemäßen Verfahren zur Herstellung einer modifizierten Stärke zu einem prebiotioschen Effekt im Dickdarm, da dort das Wachstum von vorteilhaften Bakterien unterstützt wird. Zusätzlich ist bekannt, dass RS ein bevorzugtes Substrat für Butyrat produzierende Bakterien darstellt. Butyrat dient als Hauptenergiequelle von Colonozyten. Ein hoher Gehalt von Butyrat im Colon hemmt die Entstehung von Darmkrebs.

Daher betrifft ein weiterer Gegenstand der vorliegenden Erfindung Nahrungsmittel, enthaltend erfindungsgemäße Stärke oder Stärke, hergestellt nach einem erfindungsgemäßen Verfahren zur Herstellung einer modifizierten Stärke. Vorzugsweise handelt es sich bei dem erfindungsgemäßen Nahrungsmitteln um Backwaren (z.B. Brot, Brötchen, Gebäck, Kuchen, Waffeln), Getreideprodukte (z.B. Mehl, Nudeln, Müsliriegel), Süßwaren (z.B. Schokolade, Konfekt, Speiseeis, Weingummi, Bonbons, Süßspeisen), Fleischwaren und Fleischprodukte (z.B. Fleischbrühe, Fleischsuppen), Wurstwaren (z.B. Wurstsalat, Grütze, Gelee), Milchprodukte (z.B. Joghurt, Molke, Milchgetränke), Gemüseprodukte (z.B. Gemüsebrühe, Gemüsesuppen, Salate), Kartoffelprodukte (z.B. Kartoffelsalat, Chips, Pommes frites, Kartoffelpürree).

Unter dem Begriff "Nahrungsmittel (Lebensmittel)" sollen im Zusammenhang mit der vorliegenden Erfindung alle Stoffe und Produkte, die, ggf. nach vorheriger Zubereitung, vom Menschen zum Zwecke der Ernährung und/oder des Genusses durch den Mund aufgenommen werden, verstanden werden. Dazu gehören Nahrungsmittel, Genussmittel, Lebensmittelzusatzstoffe und Nahrungsergänzungsmittel.

Zu Nahrungsmitteln zählen z.B. auch Getränke, Kaugummi sowie alle Stoffe, einschließlich Wasser, die dem Nahrungsmittel bei seiner Herstellung, Bearbeitung oder Verarbeitung absichtlich zugesetzt werden.

Nicht als Nahrungsmittel im Sinne der vorliegenden Erfindung sind Futtermittel (im Sinne der Verordnung (EG) Nr. 178/2002, 28. Januar 2002), Pflanzen vor dem Ernten, Arzneimittel (im Sinne der Richtlinien 65/65/EWG, 09.02.1965 einschließlich der Änderungen in 93/39/EWG, 24.08.1993 und 92/73/EWG, 13.10.1992) Tabak (im Sinne der Richtlinien 89/622/EWG, 08.12.1989 einschließlich der Änderungen in 92/41/EWG, 11.06.1992) und Betäubungsmittel und psychotrophe Stoffe (im Sinne des Einheitsübereinkommens der Vereinten Nationen über Suchtstoffe, 1961, und des Übereinkommens der Vereinten Nationen über psychotrophe Stoffe, 1971) anzusehen.

Weiterhin ist Gegenstand der vorliegenden Erfindung ein Verfahren zur Herstellung eines Nahrungsmittels, worin erfindungsgemäße Stärke oder Stärke, hergestellt nach einem erfindungsgemäßen Verfahren zur Herstellung einer modifizierten Stärke zu einem Nahrungsmittel hinzu gegeben wird.

Ein weiterer Gegenstand der vorliegenden Erfindung betrifft die Verwendung von erfindungsgemäßer Stärke oder Stärke, hergestellt nach einem erfindungsgemäßen Verfahren zur Herstellung einer modifizierten Stärke zur Herstellung eines Nahrungemittels, vorzugsweise eines Nahrungsmittels mit reduziertem Kaloriengehalt. Bevorzugt ist die Verwendung von erfindungsgemäßer Stärke oder Stärke, hergestellt nach einem erfindungsgemäßen Verfahren zur Herstellung einer modifizierten Stärke für die Herstellung eines Nahrungsmittels, das für die Ernährung von Diabetikern geeignet ist.

Genetisch modifizierte Pflanzen, in die ein fremdes Nucleinsäuremolekül codierend ein Protein mit der Aktivität einer GFAT eingeführt wurde oder Teile dieser Pflanzen zeichnen sich dadurch aus, dass sie eine Stärke synthetisieren, die die bereits beschriebenen Eigenschaften aufweist. Solche Pflanzen zeichnen sich vorzugsweise weiterhin dadurch aus, dass sie einen erhöhten Gehalt an N-acetylierten Glucosamin Derivaten aufweisen. N-acetyl Glucosamin hat einen stimulierenden Effekt auf das Wachstum von Bifidobakterien (Liepke et al., 2002, Eur. J. Biochem. 269, 712-718). Weiterhin wurde gezeigt, dass N-acetyl Glucosamin Lactobacillen (z.B. *Lactobacillus casei* Subspezies *paracasei*) aus Fischdärmen als Substrat dient (Adolfo Bucio Galindo, 2004, Proefschrift, Wageningen Universiteit, ISBN 90-5808-943-6). N-acetyl Glucosamin hat daher einen positiven Effekt auf probiotische Bakterien. Pflanzen, die erhöhte Gehalte an N-acetyl Glucosamin aufweisen, sollten einen positiven Effekt auf das Wachstum probiotischer Bakterien haben. Weiterhin werden N-acetylierten Glucosamin Derivate z.B. als Nahrungsergänzungsmittel vertrieben, die der Ausbildung von Gelenkerkrankungen (z.B. Arthrosen) vorbeugend entgegenwirken wirken sollen.

Genetisch modifizierte Pflanzen, in die ein fremdes Nucleinsäuremolekül codierend ein Protein mit der Aktivität einer GFAT eingeführt wurde oder Teile dieser Pflanzen zeichnen sich daher einerseits dadurch aus, dass sie eine Stärke mit erhöhtem RS Anteil synthetisieren, welche beim Verzehr einen positiven Einfluss auf die Darmflora hat. Die zusätzlich vorzugsweise in besagten Pflanzen synthetisierten N-acetylierten Glucosamin Derivate können einerseits einen weiteren positiven Effekt auf die Darmflora des Konsumenten haben aber durch kontinuierlichen Verzehr auch eine Vorbeugende Wirkung zur Verhinderung von Gelenkerkrankungen bewirken.

Besagte Pflanzen oder deren Teile, sind daher einerseits besonders geeignet für die Zubereitung von Nahrungsmitteln, die einen reduzierten Kaloriengehalt aufweisen. Andererseits können sie wie bereits ausgeführt, einen positiven Effekt auf die Magen-Darm Flora des Konsumenten haben und/oder der Ausbildung von Gelenkerkrankungen vorbeugen.

Weiterhin zeichnet sich N-acetyl-Glucosamin dadurch aus, dass es einen frischen, süßen Geschmack aufweist, wohingegen z.B. Glucosamin bitter schmeckt. Genetisch modifizierte Pflanzen, in die ein fremdes Nucleinsäuremolekül codierend ein Protein mit der Aktivität einer GFAT eingeführt wurde oder Teile dieser Pflanzen zeichnen sich daher zusätzlich dadurch aus, dass sie N-acetyl-Glucosamin Derivate enthalten, die Süßkraft vermitteln. Gegenüber herkömmlichen Süßkraft vermittelnden Substanzen (z.B. Saccharose) bieten besagte Pflanzen oder Teile dieser Pflanzen den weiteren Vorteil, dass sie beim Konsumenten keine negativen Effekte, wie z.B. Bildung von Karies oder Erzeugung eines erhöhten glykämischen Index bewirken.

Daher betrifft ein weiterer Gegenstand der vorliegenden Erfindung Nahrungsmittel, enthaltend genetisch modifizierte Pflanzenzellen oder Teile von genetisch modifizierten Pflanzen, die ein fremdes Nucleinsäuremolekül, codierend ein Protein mit der Aktivität einer Glutamin:Fructose-6-Phosphat Amidotransferase (GFAT) aufweisen, wobei besagte Pflanzenzellen oder Pflanzen eine modifizierte Stärke synthetisieren und/oder einen erhöhten Gehalt an N-acetylierten Glucosamin Derivaten aufweisen, im Vergleich zu entsprechenden Wildtyp-Pflanzenzellen bzw. Wildtyp-Pflanzen.

Vorzugsweise enthalten erfindungsgemäße Nahrungsmittel eine resistente Stärke, die die Eigenschaften von Stärke, hergestellt nach einem erfindungsgemäßen Verfahren zur Herstellung einer modifizierten Stärke aufweisen.

Unter dem Begriff "N-acetylierte Glucosamin Derivate" sind im Zusammenhang mit der vorliegenden Erfindung alle Derivate des Glucosamins (2-Amino-2-desoxy-Glucose), wozu auch Epimere wie z.B. Galactosamin (2-Amino-2-desoxy-Galactose) oder Mannosamin (2-Amino-2-desoxy-Mannose) zu zählen sind, zu verstehen, die nach der unter allgemeine Methoden Punkt 3 beschriebenen Methode gemessen werden. Bevorzugt handelt es sich bei den N-acetylierten Glucosamin Derivaten um N-acetyl Glucosamin-Phosphat (N-acetyl Glucosamin-1-Phosphat und/oder N-acetyl Glucosamin-6-Phosphat), N-acetyl Glucosamin und/oder UDP-N-acetyl Glucosamin.

N-acetylierte Glucosamin Derivate können mit dem Fachmann bekannten Methoden nachgewiesen werden (Morgan und Elson (1934, Biochem J. 28(3), 988-995). Bevorzugt wird im Zusammenhang mit der vorliegenden Erfindung die unter Allgemeine Methoden Punkt 3 beschrieben Methode zur Bestimmung des Gehaltes an N-acetylierten Glucosamin Derivaten verwendet.

Bevorzugt weisen erfindungsgemäße Nahrungsmittel auch erhöhte Mnegen an Glucosamin-Phosphat (Glucosamin-1-Phosphat und/oder Glucosamin-6-Phosphat) auf. Diese Substanzen könne vom Fachmann mittels bekannter Methoden, z.B. mit Hilfe der Massenspektroskopie nachgewiesen werden.

Unter dem Begriff "Teile von Pflanzen" sollen im Zusammenhang mit der vorliegenden Erfindung z.B.verarbeitbare Teile von Pflanzen verstanden werden, die Verwendung in der Herstellung von Nahrungsrmitteln finden, die als Rohstoffquelle für industrielle Prozesse (z.B. für die Isolierung von Stärke), als Rohstoffquelle für die Herstellung pharmazeutischer oder als Rohstoffquelle für die Herstellung kosmetischer Produkte (z.B. zur Isolierung von N-acetylierten Glucosamin Derivaten) eingesetzt werden.

Weiterhin sollen im Zusammenhang mit der vorliegenden Erfindung unter dem Begriff "Teile von Pflanzen" z.B."konsumierbare Pflanzenteile verstanden werden, die dem Menschen als Nahrungsmittel dienen.

Bevorzugte "Teile von Pflanzen" sind Früchte, Speicherwurzeln, Wurzeln, Blüten, Knospen, Sprosse, Blätter oder Stämme, besonders bevorzugt Samen, Früchte, Körner oder Knollen.

Bevorzugt enthalten erfindungsgemäße Nahrungsmittel zusätzlich zu erfindungsgemäßer modifizierter Stärke, vorzugsweise mindestens 0,05%, bevorzugt mindestens 0,1%, besonders bevorzugt mindestens 0,5%, insbesondere bevorzugt mindestens 1,0% N-acetylierte Glucosamin Derivate pro Gramm Trockengewicht.

Vorzugsweise enthalten erfindungsgemäße Zusammensetzungen zusätzlich zu erfindungsgemäßer modifizierter Stärke höchstens 10%, bevorzugt höchstens 5%, besonders bevorzugt höchstens 3%, insbesondere bevorzugt höchstens 2% N-acetylierte Glucosamin Derivate pro Gramm Trockengewicht.

Gegenüber dem Stand der Technik bieten erfindungsgemäße Nahrungsmittel, enthaltend genetisch modifizierte Pflanzenzellen oder Teile von genetisch modifizierten Pflanzen, die ein fremdes Nucleinsäuremolekül, codierend ein Protein mit der Aktivität einer Glutamin:Fructose-6-Phosphat Amidotransferase (GFAT) aufweisen, den Vorteil, dass sie selbst nach Erhitzen, was zu einer Verkleisterung der Stärke führen kann, noch einen erhöhten Gehalt as RS und SDS aufweisen. Daher eignen sich genetisch modifizierte Pflanzenzellen oder Teile von genetisch modifizierten Pflanzen, die ein fremdes Nucleinsäuremolekül, codierend ein Protein mit der Aktivität einer Glutamin:Fructose-6-Phosphat Amidotransferase (GFAT) aufweisen, besonders für die Herstellung von Nahrungsmitteln, die für ihre Herstellung erhitzt werden müssen.

Daher betrifft ein weiterer Gegenstand erfindungsgemäße Nahrungsmittel, die bei der Zubereitung erhitzt werden. Hierzu sind z.B. Konserven, aber auch gekochte Gemüse- (z.B. Süßmais, Suppen) und Kartoffelprodukte (z.B. gekochte Kartoffeln, Kartoffelsalat, Chips, Pommes frites) zu zählen.

Weiterhin sind Verfahren zur Herstellung eines erfindungsgemäßen Nahrungsmittels Gegenstand der vorliegenden Erfindung, worin genetisch modifizierte Pflanzenzellen oder genetisch modifizierte Pflanzen oder Teile von genetisch modifizierten Pflanzen, die ein fremdes Nucleinsäuremolekül codierend ein Protein mit der Aktivität einer Glutamin:Fructose-6-Phosphat Amidotransferase aufweisen, zur Herstellung des Nahrungsmittels verwendet werden.

Vorzugsweise dienen erfindungsgemäße Verfahren zur Herstellung eines Nahrungsmittels zur Herstellung eines erfindungsgemäßen Nahrungsmittels oder zur Herstellung eines Nahrungsmittels, das die Eigenschaften eines erfindungsgemäßen Nahrungsmittels aufweist.

Ein weitere Gegenstand der vorliegenden Erfindung betrifft die Verwendung von genetisch modifizierten Pflanzenzellen, genetisch modifizierten Pflanzen, Teilen von genetisch modifizierten Pflanzen, die ein fremdes Nucleinsäuremolekül codierend ein Protein mit der Aktivität einer Glutamin:Fructose-6-Phosphat Amidotransferase aufweisen zur Herstellung eines Nahrungsmittels. Vorzugsweise handelt es sich bei der Verwendung zur Herstellung eines Nahrungsmittels um die Herstellung eines Nahrungsmittels, das einen reduzierten Kaloriengehalt aufweist und/oder einen erhöhten RS Anteil aufweist und/oder das zur Vorbeugung von Gelenkserkrankungen dient und/oder das einen positiven (prebiotischen) Effekt auf die Darmflora des Konsumenten bewirkt.

## Beschreibung der Sequenzen

SEQ ID NO 1: Nucleinsäuresequenz, codierend ein Protein mit der Aktivität einer bakteriellen GFAT aus *Escherichia coli.*

SEQ ID NO 2: Aminosäuresequenz eines Proteins mit der Aktivität einer GFAT aus *Escherichia coli.* Die dargestellte Aminosäuresequenz kann von SEQ ID NO 1 abgeleitet werden.

SEQ ID NO 3: Synthetische Nucleinsäuresequenz, codierend ein Protein mit der Aktivität einer GFAT aus *Escherichia coli.* Die Synthese der Codons der dargestellten Sequenz erfolgte in der Weise, dass sie an die Verwendung von Codons in Pflanzenzellen angepasst ist. Die dargestellte Nucleinsäuresequenz codiert ein Protein mit der unter SEQ ID No 2 dargestellten Aminosäuresequenz.

SEQ ID NO 4: Nucleinsäuresequenz, codierend ein Protein mit der Aktivität einer GFAT-2 aus Maus.

SEQ ID NO 5: Aminosäuresequenz eines Proteins mit der Aktivität einer GFAT-2 aus Maus. Die dargestellte Aminosäuresequenz kann von SEQ ID NO 4 abgeleitet werden.

## Beschreibung der Abbildungen

FIG 1: Zeigt den Gehalt an mittels Lugol'scher Lösung färbbarer Stärke in gekochten Scheiben von Kartoffelknollen nach Verdau mit Pankreatin (siehe Beispiel 12).

Der Inhalt aller zitierten Veröffentlichungen, inklusive der betreffend für Sequenz Datenbanken genannter "Accession Numbers" von Nucleinsäuremolekülen und Aminosäuresequenzen ist durch die Nennung des Zitates in die Beschreibung der Anmeldung mit aufgenommen.

Im Folgenden werden Methoden beschrieben, die im Zusammenhang mit der vorliegenden Erfindung verwendet werden können. Diese Methoden stellen konkrete Ausführungsformen dar, beschränken die vorliegende Erfindung jedoch nicht auf diese Methoden. Dem Fachmann ist bekannt, dass er durch Modifikation der beschriebenen Methoden und/oder durch Ersetzen einzelner Methoden bzw. Methodenteile durch alternative Methoden bzw. alternative Methodenteile die Erfindung in gleicher Weise ausführen kann.

**Allgemeine Methoden**

1. Transformation von Kartoffelpflanzen

Kartoffelpflanzen wurden mit Hilfe von *Agrobacterium,* wie bei Rocha-Sosa et al. (EMBO J. 8, (1989), 23-29) beschrieben, transferiert.

2. Extraktion von Stärke aus Kartoffelpflanzen

Kartoffelknollen werden in einem handelsüblichen Entsafter (Multipress automatic MP80, Braun) aufgearbeitet. Das stärkehaltige Fruchtwasser wird in ein Gefäß, in dem etwas Leitungswasser enthaltend Natrium- Disulfit vorgelegt wurde, aufgefangen. Im Anschluss wird das Gefäß mit Leitungswasser vollständig aufgefüllt. Nach etwa 2 stündigem Absetzen der Stärke wird der Überstand dekantiert, die Stärke erneut in Leitungswasser aufgeschwemmt und über ein Sieb mit 125 µm Maschenweite gegeben. Nach etwa 2 Stunden (Stärke hat sich erneut am Boden des Eimers abgesetzt) wird erneut der wässrige Überstand dekantiert. Dieser Waschvorgang wird noch dreimal wiederholt, so dass die Stärke insgesamt fünfmal in frischem Leitungswasser resuspendiert wird. Im Anschluss werden die Stärken bei 37 °C auf einen Wassergehalt von 12% bis 17% getrocknet und im Mörser homogenisiert. Die Stärken stehen nun für Analysen zu Verfügung.
Typische Mengenverhältnisse für die beschriebene Aufarbeitungsmetohde: Stärkehaltiges Fruchtwasser von 4 bis 5 kg Kartoffelknollen wird in einem 10 Liter Gefäß in das ca. 200 ml Leitungswasser enthaltend 3 bis 4 g Natrium-Disulfit vorgelegt wurde, überführt. Vor dem Absetzten der Stärke wird das Volumen jeweils auf ca. 10 Liter aufgefüllt und die Stärke resuspendiert. Je nach Menge eingesetzter Kartoffelknollen, können die angegebenen Mengen entsprechend angepasst werden.

3. Bestimmung des Gehaltes an N-acetylierten Glucosaminen

N-acetylierte Glucosamin Derivate, die ein reduzierendes Ende aufweisen, wurden in Anlehnung an die Methode von Elson und Morgan (1933, J Biochem. 27,1824) und der von Reissig et al. (1955, Biol. Chem. 217, 959-966) verbesserten colorimetrischen Bestimmungsmethode durchgeführt. Die colorimetrische Bestimmungsmethode beruht auf einer Reaktion von Chromogen III (Muckenschnabel et al., 1998, Cancer Letters 131, 13-20) mit p-dimethylaminobenzaldehyd (DMAB, Ehrlich's Reagenz) zu einem rot gefärbten Produkt, dessen Konzentration photometrisch bestimmt werden kann.

a) Aufarbeitung des Pflanzenmaterials

Zunächst wurde geerntetes Pflanzenmaterial zerkleinert. Je nach verwendeter Menge des Pflanzenmaterials erfolgte die Zerkleinerung in einer Labor-Schwingkugelmühle (MM200, Firma Retsch, Germany) für 30 Sekundenb bei 30 HZ oder mittels eines Warring Blendors für ca. 30 Sekunden bei maximaler Geschwindigkeit. In der Regel wurden ca. 0,5 g des zerkleinerten Pflanzenmaterials (z. B. Blatt, Knolle oder Reiskorn) mit 1 ml einer Lösung bestehend aus 7%-Perchlorsäure, 5 mM EGTA versetzt gemischt und für 20 Minuten auf Eis inkubiert. Anschließend erfolgte eine Zentrifugation (5 Minuten bei 16000xg, 4°C). Der nach Zentrifugation erhaltene Überstand wurde abgenommen und mit einer Lösung bestehend aus 5M KOH, 1M TEA neutralisiert (auf pH 7,0 eingestellt), bevor nochmals eine Zentrifugation (5 min bei 16000xg, 4°C) erfolgte. Nach erfolgter Zentrifugation wurde der Überstand abgenommen, dessen Volumen bestimmt und mit der unter b) beschriebenen Methode die Menge an N-acetyierten Glucosamin Derivaten, die ein reduzierendes Ende aufweisen, bestimmt.

b) Bestimmung des Gehaltes an N-acetylierten Glucosamin Derivaten mit reduzierendem Enden

Zu 100 µl nach der unter a) beschrieben Methode erhaltenem Pflanzenextrakt werden 20 µl einer Lösung bestehend aus 0,8 M $K_2B_4O_7$, pH 9,6 gegeben und nach gutem Durchmischen für 5 min auf 95°C erhitzt. Nach Abkühlung des Gemisches auf Raumtemperatur wird dem Gemisch 0,7 ml Ehrlichs Reagenz (mit Eisessig 1:10 verdünnte Lösung bestehend aus 10g DMAB in 12,5 ml HCl konz., 87,5 ml Eisessig) zugegeben, erneut gemischt und für weitere 30 Minuten bei 37°C inkubiert. Anschließend erfolgt eine Zentrifugation des Gemisches für 1 Minute bei 16000xg, bevor die optische Dichte (OD) des nach Zentrifugation erhaltenen Überstandes im Photometer bei 585 nm bestimmt wird.

c) Berechnung der Konzentration an N-acetylierten Glucosamin Derivaten

Zunächst wurde eine Eichkurve unter Verwendung von definierten Mengen N-acetyl-Glucosamin-6-Phosphat erstellt. Dazu wurde die OD von Lösungen enthaltend 0 mM, 0,1 mM, 0,5 mM, 1 mMA, 5 mM und 10 mM N-acetyl-Glucosamin-6-Phosphat nach der unter b) beschriebenen Methode bestimmt
Die Erstellung der Eichgerade erfolgte in Microsoft Excel, indem durch die für die einzelnen Konzentrationen erhaltenen Messpunkte eine polynomische Trend/Regressionslinie 2. Ordnung nach der Formel

$$y = ax^2 + bx + c \text{ bzw. } y = x^2 + px + q$$

gelegt wurde. Die erhaltene Gleichung wurde für die Berechnung der Werte nach x aufgelöst so dass sich ergibt:

$$x = -p/2 - \text{Wurzel } (p^2 / 4 - q)$$

wobei
p = b / a, q = (c-y) / a und y die gemessene OD der unbekannten Probe ist.
Unter Berücksichtigung des eingesetzten Frischgewichts, eingesetzten Volumens und unter Berücksichtigung eines eventuell verwendeten Verdünnungsfaktors wurden die Gehalte in μmol (in der Messlösung) bzw. in μmol pro g Frischgewicht berechnet.

4. Bestimmung der Aktivität einer GFAT

Die Bestimmung der Aktivität eines Proteins mit der Aktivität einer GFAT wird wie bei Rachel et al. (1996, J. Bacteriol. 178 (8), 2320-2327) beschrieben durchgeführt. Zur Unterscheidung ob ein Protein die Aktivität einer GFAT-1 oder GFAT-2 aufweist, wird die bei Hu et al. (2004, J. Biol. Chem. 279 (29), 29988-29993) beschriebene Methode verwendet.

5. Nachweis von N-acetylierten Glucosamin Derivaten mittels Massenspektroskopie

Zum Nachweis von N-acetylierten Glucosaminderivaten mittels Massenspektroskopie wurde pflanzliches Gewebe wie unter Allgemeine Methoden Punkt 4 a) aufgearbeitet. Um einen möglichst salzfreien Extrakt zu erhalten, wurden die jeweiligen Proben vor der massenspektroskopischen Untersuchung zunächst bei -20°C eingefroren und während einer Zentrifugation (16000xg bei Raumtemperatur) aufgetaut. Der Überstand wurde für die Messung 1:20 mit einem Methanol : Wasser Gemisch im Verhältnis 1:1 (Volumen/Volumen) verdünnt.
Es wurden MS Spektren mit drei verschiedenen Detektor Empfindlichkeiten aufgenommen um die Nachweisempfindlichkeit für schwache Signale (Peaks) zu erhöhen. Das Ansprechverhalten ("Response") des Detektors ist in diesem Fall jedoch nicht mehr linear was beim Vergleich der Signalintensitäten ("Peak"-Flächen) unterschiedlicher Metabolite auffällt und berücksichtigt werden sollte. Um die Vergleichbarkeit der Messungen zu gewährleisten, wurde darauf geachtet dass die einzelnen Proben bei gleicher Detektoreinstellung auch gleiche Signalintensitäten (in cps, counts per second) ergaben.
Die Flächen der erhaltenen Signale ("Peak"-Flächen), die den unterschiedlichen Metaboliten zugeordnet wurden, werden relativ zur Peakfläche von Hexosen (m/z=179) in % angegeben. Aus dem Verhältnis der Signalintensitäten ("Peak"-Flächen) in unterschiedlichen Proben kann eine Aussage über die Konzentrationsverhältnisse der entsprechenden N-acetylierten Glucosamin Derivate in Bezug auf die Konzentration von Hexosen in der betreffenden Probe getroffen werden.
Parallel wurden MS-MS Messungen der einzelnen Proben und von einzelnen entsprechenden Referenzsubstanzen (Glucosamin, N-acetyl Glucosamin, Glucosamin-6-Phosphat, Glucosamin-1-Phosphat, N-acetyl Glucosamin-6-Phosphat, N-acetyl Glucosamin-1-Phosphat, UDP-N-acetyl Glucosamin) durchgeführt, Dadurch kann eine Abschätzung erfolgen, ob es sich bei dem Signal ("Peak") das für die Flächenbestimmung genutzt wird, um ein Signal handelt, dass ausschließlich durch ein spezifisches Metabolit, bzw. durch spezifische isomere Metabolite mit gleicher Masse generiert wurde, oder ob das betreffende Signal nur partiell dem entsprechen Metabolit bzw. den entsprechenden spezifischen isomeren Metaboliten mit gleicher Masse zuzuordnen ist.
MS und MS-MS Spektren wurden im negativ-Modus an einem Q-STAR Pulsar i hybrid Massenspektrometer der Firma Applied Biosystems gemessen welches mit einer nano-Elektrospray Quelle ausgerüstet ist. Es wurden dabei hauptsächlich deprotonierte einfach geladene Ionen detektiert.

Die Messungen erfolgten unter folgenden Bedingungen:

| | |
|---|---|
| Massenbereich | 50-700 Da. |
| Detektorempfindlichkeit: | 2000, 2050 bzw. 2100 |

Bei jeder der drei Detektoreinstellungen wurde darauf geachtet dass für die Proben möglichst ähnliche Signalintensitäten (in cps, counts per second) erreichet wurden.

6. Stärkeanalytik

a) Bestimmung des Amylosegehaltes bzw. des Amylose/Amylopektinverhältnisses

Stärke wurde nach Standardmethoden aus Kartoffelpflanzen isoliert, und der Amylosegehalt sowie das Verhältnis Amylose zu Amylopektin wurde nach der von Hovenkamp-Hermelink et al. beschriebenen Methode (Potato Research 31, (1988), 241-246) bestimmt.

b) Bestimmung des Phosphatgehaltes in C-6-Position der Glucosemoleküle der Stärke

In der Stärke können die Positionen C2, C3 und C6 der Glucoseeinheiten phosphoryliert sein. Zur Bestimmung des C6-P-Gehaltes der Stärke werden 50 mg Stärke in 500 µl 0,7 M HCl 4 h bei 95°C hydrolysiert. Anschließend werden die Ansätze für 10 min bei 15500 g zentrifugiert und die Überstände abgenommen. Von den Überständen werden 7µl mit 193 µl Imidazol-Puffer (100 mM Imidazol, pH 7,4; 5 mM $MgCl_2$, 1 mM EDTA und 0,4 mM NAD) gemischt. Die Messung wurde im Photometer bei 340 nm durchgeführt. Nach der Etablierung einer Basisabsorption wurde die Enzymreaktion durch die Zugabe von 2 Einheiten (units) Glucose-6-Phosphat Dehydrogenase (von Leuconostoc mesenteroides, Boehringer Mannheim) gestartet. Die Absorptionsänderung ist direkt proportional zur Konzentration des G-6-P Gehaltes der Stärke.

c) Bestimmung der Viskositätseigenschaften mittels eines Rapid Visco Analyzers (RVA)

2 g Stärke (TS) werden in 25 ml $H_2O$ (VE Wasser, Leitfähigkeit von mindestens 15 mega Ohm) aufgenommen und für die Analyse in einem Rapid Visco Analyser (Newport Scientific Pty Ltd., Investmet Support Group, Warriewod NSW 2102, Australien) verwendet. Der Betrieb des Gerätes erfolgt nach den Angaben des Herstellers. Dabei erfolgt die Angabe der Viskositätswerte in RVUs gemäß der Betriebsanleitung des Herstellers, die hiermit insoweit durch Bezugnahme in die Beschreibung aufgenommen wird. Zur Bestimmung der Viskosität der wäßrigen Lösung der Stärke wird die Stärkesuspension zunächst für eine Minute auf 50°C erhitzt (Schritt 1), danach von 50°C auf 95°C mit einer Geschwindigkeit von 12°C pro Minute (Schritt 2) erhitzt. Anschließend wird die Temperatur für 2.5 Min bei 95°C gehalten (Schritt 3). Danach wird die Lösung von 95°C auf 50°C abgekühlt, mit einer Geschwindigkeit von 12°C pro Minute (Schritt 4). Während der gesamten Dauer wird die Viskosität bestimmt.
Nach Beendigung des Programms wird der Rührer entfernt und der Becher abgedeckt. Die verkleisterte Stärke steht nun für die Analyse der Gelfestigkeit (siehe unten) nach 24 h zur Verfügung.
Im Profil der RVA Analyse gibt es charakteristische Werte, die zum Vergleich unterschiedlicher Messungen und Substanzen dargestellt werden. Die folgenden Begriffe sollen im Zusammenhang mit der vorliegenden Erfindung wie folgt verstanden werden:

Maximale Viskosität (RVA Max)
Unter der maximalen Viskosität versteht man den höchsten Viskositätswert, gemessen in RVUs, der in Schritt 2 oder 3 des Temperaturprofils erreicht wird.
Minimale Viskosität (RVA Min)
Unter der minimalen Viskosität versteht man den geringsten Viskositätswert, gemessen in RVUs, der nach der Maximalen Viskosität im Temeraturprofil auftritt. Normalerweise erfolgt dieses in Schritt 3 des Temperaturprofils.
Finale Viskosität (RVA Fin)
Unter der Finalen Viskosität versteht man den Viskositätswert, gemessen in RVUs, der am Ende der Messung auftritt.
Setback (RVA Set)
Der so genannte "Setback" wird berechnet, indem man den Wert der Finalen Viskososität von der desjenigen Minimums, welches im Kurvenverlauf nach erreichen der Maximalen Viskosität auftritt, subtrahiert.
Verkleisterungstemperatur (Pasting Temp)
Unter der Verkleisterungstemperatur versteht man die Temperatur im RVA Profil, bei welcher die Viskosität zum erstem mal stark innerhalb einer kurzen Periode ansteigt.
Peak Time (RVA T)
Unter der Peak Time versteht man die Zeit im Temperaturprofil, zu welcher die Viskosität den maximalen Wert erreicht hat.

d) Bestimmung der Gelfestigkeit (Texture Analyser)

2 g Stärke (TS) werden in 25 ml einer wäßrigen Suspension im RVA-Gerät verkleistert (Temperaturprogramm: siehe oben) und anschließend für 24 h bei Raumtemperatur in einem geschlossenen Gefäß gelagert. Die Proben werden unter der Sonde (zylindrischer Stempel mit planer Oberfläche) eines Texture Analysers TA-XT2 der Firma Stable Micro Systems (Surrey, UK) fixiert und die Gelfestigkeit mittels Einstellung der folgenden Parameter am Gerät bestimmt:

| | |
|---|---|
| Test-Geschwindigkeit | 0,5 mm/s |
| Eindringtiefe | 7 mm |
| Kontaktfläche | 113 $mm^2$ |
| Druck | 2 g |

e) Korngrößenbestimmung

Stärke wurde nach Standardmethoden aus Kartoffelknollen extrahiert (siehe oben). Die Korngrößenbestimmung wurde dann mit einem Laserdiffraktometer des Typs "Mastersizer 2000" der Firma Malvern Instruments Ltd. unter Verwendung der migelieferten Softwareversion 5.22 durchgeführt.
Grundlage der Laserdiffraktometrie ist die Beugung des Lichtes, wobei je nach Form und Größe des Teilchens, an dem das Licht gebeugt wird, unterschiedliche Beugungsbilder entstehen. Große Partikel erzeugen einen kleinen Beugungswinkel und kleine Partikel einen großen Beugungswinkel. An Hand der erhaltenen Beugungswinkel wird mittels des genannten Computerprogramms (Softwareversion 5.22) die Partikelgrößr ermittelt.
Für die Bestimmung der Korngröße von Kartoffelstärken wurden folgende Einstellungen verwendet:

| | |
|---|---|
| Dispergiermedium: | Wasser (Brechungsindex von 1,33) |
| eingestellte Lichtabschattung (obscuration): | 12% |
| Dispergierzeit | 1 min |
| Messbereich: | 0,02 - 2000 μm |
| Lichtquellen: | rotes Licht - Helium Neon Laser |
| blaues Licht | Halbleiter Lichtquelle |
| Meßzyklen: | 5 |
| Meßzeit:: | 6 sec. |
| Background: | 6 sec. |
| Zeit zwischen den Messungen: | 5sec. |
| Rührergeschwindigkeitt: | 3500rpm |
| Auswertungsmethode: | Fraunhofer Näherung |

f) Analyse von Stärke mittels Wärmefluss-Kalorimetrieverfahren (Differential scanning calorimetry (= DSC))

Einwaagen von etwa 10 mg (Trockengewicht) Mais- oder Weizenmehl oder Mais- oder Weizenstärke wurden mit einem Überschuss, vorzugsweise 3-fachem Überschuss an demineralisiertem Wasser (bevorzugt 20 μl) in Edelstahlpfännchen (Perkin Elmer, "Large Volume Stainless Steel Pans" [03190218], Volumen 60 μl) gegeben und mit Hilfe einer Presse hermetisch verschlossen. Mit einer Heizgeschwindigkeit von 10°C/min wurde die Probe von 20°C bis 120°C in einem Diamond DSC-Gerät (Perkin Elmer) erhitzt. Dabei dient ein leeres verschlossenes Edelstahlpfännchen als Referenz. Das System wurde mit definierten Mengen Indium bzw. Oktadekan kalibriert.
Die Datenanalyse wurde mittels Softwareprogramm von Pyris (Perkin Elmer, Version 7.0) durchgeführt. Die Weiterverarbeitung auswertbarer Rohdaten erfolgte durch Analyse der einzelnen Peaks der Phasenübergänge 1. Ordnung auf T-onset (°C), T-peak (°C), T-end (°C) und dH (J/g) (Standard ist dabei die gerade Basislinie).
DSC T-onset ist dabei charakterisiert als der Schnittpunkt zwischen der Verlängerung der Basislinie und der an die ansteigende Flanke des Peaks angelegten Tangente durch den Wendepunkt. Sie charakterisiert den Beginn der Phasenumwandlung.

Als Maximaltemperatur DSC T-peak wird die Maximaltemperatur bezeichnet, bei der die DSC-Kurve ein Maximum erreicht hat (d.h., diejenige Temperatur, bei der die erste Ableitung der Kurve Null ist).
Bei der in Pyris verwendeten Funktion (calc-peak Area) wird von Hand eine Start- und eine Endtemperatur zur Festlegung der Basislinie eingegeben.

7. Bestimmung des Anteiles an resistenter Stärke (Verdaubarkeit)

Bevor der Anteil an resistenter Stärke bestimmt wurde, wurden jeweils ca. 10 mg Stärke in 10 μl demineralisiertem Wasser suspendiert und für 10 Minuten unter Schütteln bei 90°C inkubiert. Anschließend erfolgte direkt die Bestimmung des Anteils an resistenter Stärke.
Die Bestimmung des Anteils an resistenter Stärke erfolgt nach der in Englyst et al. ((Europ. J. of Clinical Nutrition 46 (Suppl. 2), (1992), S 33-50)) beschriebenen Methode (siehe insbesondere folgende Abschnitte aus Englyst et al., Seite S35-S36: "Reagents, Apparatus, Spectrophotometer"; Seite S36-S37, Absatz "Measurement of free glucose (FG) "; Seite S38, Absatz "Measurement of RDS and SDS").
Die Methode von Englyst et al. kann alternativ, wie von Zhang et al. (Biomacromolecules 7, (2006), 3252-3258, insbesondere Seite 3253: Methods. Enzymatic Starch Hydrolysis) beschrieben, durchgeführt werden.
Im Labormaßstab kann die Methode von Englyst et al. in folgender Weise durchgeführt werden.
Zur Herstellung der Enzymlösung werden 1,2 g Pankreatin (Merck) in 8 ml Wasser für 10 Minuten bei 37°C extrahiert. Nach Zentrifugation (10', 3000 U/min; RT) werden 5,4 ml des Überstandes mit 84 U Amyloglukosidase (Sigma-Aldrich, Taufkirchen) vermischt und mit Wasser auf ein Endvolumen von 7 ml aufgefüllt.
Parallel werden 10 mg (Trockengewicht) Stärke oder bereits gelatinisierte Stärke pro Probe in einem 2 ml Reaktionsgefäß mit 0,75 ml Natrium-Acetat-Puffer (0,1 M Natrium-Acetat pH 5, 2; 4 mM $CaCl_2$) versetzt und zur Erwärmung des Ansatzes für 5 Minuten bei 37°C inkubiert.
Der Verdau der Stärke wird durch Zugabe von jeweils 0,25 ml Enzymlösung pro Ansatz gestartet. Als Kontrolle dient ein Ansatz, zu dem Wasser anstelle von Enzymlösung gegeben wird. Nach 20, 60 und 120 Minuten werden Aliquots von 100 μl entnommen und direkt in das vierfache Volumen Ethanol gegeben, wodurch die Enzyme inaktiviert werden. Diese Verdünnung wird zur Messung des Gehaltes an Glucose verwendet.
Hierzu werden 2 μl verdünnte Probe mit 200 μl Messpuffer (100 mM Imidazol/HCl pH 6,9, 5 mM $MgCl_2$, 1 mM ATP, 2 mM NADP) vermischt und die Absorption der Probe bei 340 nm erfasst. Die Umsetzung der Glucose wird durch Zugabe von 2μl Enzym-Mix (10 μl Hexokinase, 10 μl Glucose-6-Phosphat-Dehydrogenase, 80 μl Messpuffer) gestartet und der äquimolare Umsatz von NADP zu NADPH bei 340 nm bis zum Erreichen eines Plateaus verfolgt. Die ermittelten Glucose-Mengen werden in Relation zur Einwaage gesetzt und ergeben den Anteil der Probe, der nach dem entsprechenden Zeitraum als Glucose freigesetzt wurde.

## Beispiele

1. Erwerb einer Nucleinsäuresequenzen, codierend für Protein mit der Aktivität einer GFAT-2 aus Maus

Die Nucleinsäuresequenz, codierend ein Protein mit der Aktivität einer GFAT-2 (Glutamin-Fructose-6-Phosphate Amidotransferase oder Glucosamin-6-Phosphate Synthase, EC 2.6.1.16), wurde von der Firma Invitrogen (Clone ID 4167189, cDNA clone MGC:18324, IMAGE:4167189) erworben. Hierbei handelt es sich um einen Klon der vom I.M.A.G.E. Konsortium (http://image.llnl.gov) hergestellt und Invitrogen vertrieben wird. Die cDNA codierend ein Protein mit der Aktivität einer GFAT-2 wurde in den Vektor pCMV Sport 6 der Firma Invitrogen kloniert. Das Plasmid wurde mit IC 369-256 bezeichnet. Die Nucleinsäuresequenz codierend das Protein mit der Aktivität einer GFAT-2 aus *Mus musculus,* ist unter SEQ ID NO 4 dargestellt.

2. Synthese der Nucleinsäuresequenzen, codierend für ein Protein mit der Aktivität einer bakteriellen GFAT aus *Escherichia coli*

Die Nucleinsäuresequenz, codierend ein Protein mit der Aktivität einer bakteriellen GFAT (Glutamin-Fructose-6-Phosphate Amidotransferase oder Glucosamin-6-Phosphate Synthase, *glms,* EC 2.6.1.16) aus *Escherichia coli* wurde von der Firma Entelechon GmbH synthetisiert und in den Vektor pCR4Topo der Firma Invitrogen (Prod. Nr. K4510-20) kloniert. Das erhaltene Plasmid wurde mit IC 373-256 bezeichnet. Die synthetische Nucleinsäuresequenz codierend das Protein mit der Aktivität einer bakteriellen GFAT aus *Escherichia coli,* ist unter SEQ ID NO 3 dargestellt. Die korrespondierende, ursprünglich aus *Escherichia coli* isolierte Nucleinsäuresequenz, ist unter SEQ ID NO 1 dargestellt.

3. Herstellung des Plasmides pBinB33

Der Promotor des Patatin Gens B33 aus *Solanum tuberosum* (Rocha-Sosa et al., 1989, EMBO J. 8, 23-29) wurde als *Dra* / Fragment (Nucleotide -1512 bis +14) in den mit *SST* / geschnittenmen Vektor pUC19, dessen Enden mit Hilfe von T4-DNA Ligase geglättet worden waren, ligiert. Daraus entstand das Plasmid pUC19-B33. Aus diesem Plasmid wurde der B33 Promotor mit den Restriktionsendonucleasen *Eco RI* und *Sma* / herausgeschnitten und in den entsprechend geschnittenen Vektor pBinAR (Höfgen und Willmitzer, 1990, Plant Science 66, 221-230) ligiert. Der daraus hervorgehende Vektor wurde mit pBinB33 bezeichnet.

4. Herstellung des Plasmides pBinB33Hyg

Ausgehend von pBinB33 wurde ein Fragment umfassend den B33-Promotor, einen Teil des Polylinkers sowie den ocs-Terminator mittels der Restriktionsendonukleasen das *EcoRI* und *HindIII* herausgeschnitten und in den entsprechend geschnittenen Vektor pBIB-Hyg (Becker, 1990, Nucleic Acids Res. 18, 203) kloniert. Das erhaltene Plasmid wurde mit pBinB33Hyg bezeichnet.

5. Herstellung des pflanzlichen Expressionsvektors IC 398-311, enthaltend eine codierende Nucleinsäuresequenz für ein Protein mit der Aktivität einer bakteriellen GFAT

Aus dem Plasmid IC 373-256 wurde mittels Restriktionsverdau mit *Ecl 136* I und *Xho* I die codierende Sequenz des Proteins mit der Aktivität einer bakteriellen GFAT aus *E. coli* isoliert und in die *Sma* I und *Sal* I Schnittstellen des Vektors pBinB33Hyg ligiert. Der erhaltene pflanzliche Expressionsvektor wurde mit IC 398-311 bezeichnet.

6. Kartoffelpflanzen, die ein Nucleinsäuremolekül, codierend ein Protein mit der Aktivität einer bakteriellen GFAT aufweisen

a) Tranformation von Kartoffelpflanzen

Kartoffelpflanzen (Kultivar Désirée) wurden mit dem pflanzlichen Expressionsvektor IC 398-311, enthaltend eine codierende Nucleinsäuresequenz für ein Protein mit der Aktivität einer bakteriellen GFAT aus *Escherichia coli* unter der Kontrolle des Promotors des Patatin Gens B33 aus *Solanum tuberosum* (Rocha-Sosa et al., 1989, EMBO J. 8, 23-29) nach der unter Allgemeine Methoden Punkt 1 angegebnen Methode transformiert. Die erhaltenen transgenen Linien, die mit dem Plasmid IC 398-311 transformiert sind, wurden mit 432 ES bezeichnet.

b) Analyse der Linien 432 ES

Pflanzen der Linie 432 ES wurden im Gewächshaus in 6 cm Töpfen in Erde kultiviert. Ca. jeweils 0,3 g bis 0,8 g Blattmaterial, geerntet von einzelnen Pflanzen wurden nach der unter Allgemeine Methoden Punkt 3 beschriebenen Methode aufgearbeitet und der Gehalt an N-acetylierten Glucosamin Derivaten bestimmt. Für einzelne Pflanzen mit erhöhtem Gehalt an N-acetylierten Glucosaminderviaten wurden folgende Ergebnisse erhalten:

| Pflanze | Blatt μmol/g FG | Knolle μmol/g FG |
|---|---|---|
| wt | 0,17 | 0,0 |
| 432ES 9 | 11,48 | 14,1 |
| 432ES 22 | 9,73 | 20,8 |
| 432ES 40 | 8,69 | 12,4 |

Tabelle 1: Menge an N-acetylierten Glucosamin Derivaten (in μmol pro Gramm Frischgewicht), die in Blättern bzw. Knollen von unabhängigen, transgenen Pflanzen der Linie 432 ES gemessen wurde. Spalte 1 bezeichnet jeweils die unabhängig aus der Transformation hervorgegangene Pflanze, von welcher Material geerntet wurde (wt bezeichnet hierbei entsprechende Wildtyp-Pflanzen, die nicht transformiert sind).

7. Bestimmung des Anteils an resistenter Stärke in Pflanzen der Linie 432ES

Pflanzen der Linie 432 ES wurden im Gewächshaus in Töpfen in Erde kultiviert. Reife Knollen dieser Linien wurden geerntet. Anschließend wurde Stärke nach der unter Allgemeine Methoden, Punkt 2 beschriebenen Methode isoliert und der Anteil an RDS, SDS bzw. RS in der jeweiligen Stärke nach der unter Allgemeine Methoden, Punkt 7 beschriebenen Methode bestimmt.
Die in der folgenden Tabelle dargestellten Werte wurden wie folgt ermittelt:

RDS: Menge der aus Stärke freigesetzten Glucose nach 20 Minuten Verdau (Glc 20 Min) in Gewichtsprozent [%] bezogen auf die Menge des Trockengewichtes der für den Verdau eingesetzten Stärke (TG):

$$\text{RDS [\%]} = \text{Glc 20 Min / TG x 100}$$

SDS: Differenz der Menge der aus der Stärke freigesetzten Glucose nach 120 Minuten Verdau (Glc 120 Min) und der Menge der aus Stärke freigesetzten Glucose nach 20 Minuten Verdau (Glc 20 Min), jeweils in Gewichtsprozent [%] bezogen auf die Menge des Trockengewichtes [TG] der für den Verdau eingesetzten Stärke:

$$\text{SDS [\%]} = \text{Glc 120 Min[\%]} - \text{Glc 20 Min [\%]}$$

RS: Differenz der Menge der für den Verdau eingesetzten Stärke bezogen auf das Trockengewicht in Prozent (=100%) und der Menge der aus der Stärke freigesetzten Glucose nach 120 Minuten Verdau in Gewichtsprozent [%] bezogen auf die Menge des Trockengewichtes [TG] der für den Verdau eingesetzten Stärke:

$$\text{RS [\%]} = \text{100\% - Glc 120 Min [\%]}$$

Zur Ermittlung der in der folgenden Tabelle dargestellten Werte für RDS (nach 20 Minuten und "nach 120 Minuten wurden je 3 unabhängige Proben der betreffenden Stärken mit jeweils 2 Wiederholungen vermessen und der jeweilige Mittelwert ermittelt. Die Standardabweichung (Stabw) wurde nach der allgemeinen Formel:

$$\text{Stabw} = \text{Wurzel} \left[(n\Sigma x^2 - (\Sigma x)^2) / n(n-1)\right]$$

ermittelt:
Es wurden folgende Ergebnisse erhalten:

| | Aus Stärke freigesetzte Glucose in % bezogen auf TG | | | | | |
|---|---|---|---|---|---|---|
| | RDS (nach 20 Minuten) | Stabw | nach 120 Minuten | Stabw | SDS | RS |
| wt | 19 | 6 | 66 | 9 | 47 | 34 |
| 432ES9 | 15 | 5 | 57 | 6 | 42 | 43 |
| 432ES22 | 15 | 4 | 40 | 8 | 25 | 60 |
| 432ES40 | 17 | 1 | 56 | 2 | 39 | 44 |
| Novelose 330 | 34 | 1 | 51 | 0 | 17 | 49 |

Tabelle 2: Menge an RDS, SDS und RS in Stärken, isoliert aus Knollen der Pflanzen 432ES22, 432ES9 und 432ES 40, sowie entsprechenden nicht transformierten Wildty-Pflanzen (wt). Novelose® 330 bezeichnet ein käufliches RS Produkt der Firma National Starch.

8. Bestimmung der Thermostabilität von Stärken, isoliert aus Pflanzen der Linie 432ES mittels DSC Analyse

Stärken von unabhängigen Pflanzen der Linie 432ES wurden wie unter Beispiel 7 beschrieben hergestellt und anschließend nach der unter Allgemeine Methoden, Punkt 6 f) beschrieben Methode, analysiert. Zur Ermittlung der in der folgenden Tabelle dargestellten Werte wurden je 2 unabhängige Proben der betreffenden Stärken vermessen, die Mittelwerte ermittelt und die Standardabweichung (Stabw) nach der unter Beispiel 6 b) dargestellten Formel ermittelt.

Es wurden folgende Ergebnisse erhalten:

| | To [°C] | Stabw | Tp [°C] | StAbw | Te - To | StAbw | dH [J/g] | StAbw |
|---|---|---|---|---|---|---|---|---|
| wt | 66,60 | 0,02 | 70,02 | 0,05 | 9,40 | 0,06 | 21,21 | 0,39 |
| 432ES09 | 67,65 | 0,43 | 71,01 | 0,57 | 10,41 | 0,04 | 21,30 | 0,55 |
| 432ES22 | 69,42 | 0,18 | 73,59 | 0,08 | 10,20 | 0,13 | 21,26 | 0,36 |
| 432ES40 | 67,62 | 0,11 | 71,08 | 0,11 | 10,16 | 0,41 | 22,49 | 1,51 |

Tabelle 3: Bestimmung der Thermostabilität von Stärken, isoliert aus den Pflanzen 432ES09, 432ES33, 432ES40 und entsprechenden nicht transformierten Wildrtyp-Pflanzen (wt) mittels DSC Analyse.

In einer weiteren Analyse wurden Stärken, die nach Verkleisterung in einer ersten DSC Analyse für 7 Tage bei Raumtemperatur in den verschlossenen Edelstahlpfännchen gelagert (retrogradiert) wurden, nochmals mittels DSC analysiert. In der Tabelle sind die Mittelwerte von jeweils 2 unabhängigen Proben der betreffenden Stärken dargestellt. Die Standardabweichung (Stabw) nach der unter Beispiel 6 b) dargestellten Formel ermittelt.
Es wurden folgende Ergebnisse erhalten:

| | To [°C] | Stabw | Tp [°C] | Stabw | Te - To | Stabw | dH [J/g] | Stabw |
|---|---|---|---|---|---|---|---|---|
| wt | 53,54 | 0,01 | 69,68 | 1,91 | 26,06 | 0,33 | 6,07 | 0,84 |
| 432ES09 | 54,96 | 2,81 | 69,84 | 1,14 | 26,27 | 2,43 | 7,88 | 0,31 |
| 432ES22 | 52,41 | 0,71 | 67,88 | 0,01 | 29,46 | 0,89 | 8,72 | 0,46 |
| 432ES40 | 53,04 | 0,04 | 67,86 | 0,88 | 26,79 | 0,38 | 7,16 | 0,37 |

Tabelle 4: Bestimmung der Thermostabilität von retrogradierten Stärken, isoliert aus den Pflanzen 432ES09, 432ES33, 432ES40 und entsprechenden nicht transformierten Wildrtyp-Pflanzen (wt) mittels DSC Analyse.

9. Bestimmung der Viskositätseigenschaften von Stärken, isoliert aus Pflanzen der Linie 432ES mittels RVA Analyse

Stärken von unabhängigen Pflanzen der Linie 432ES wurden wie unter Beispiel 7 beschrieben hergestellt und anschließend nach der unter Allgemeine Methoden, Punkt 6 c) beschrieben Methode, analysiert. Es wurden folgende Ergebnisse erhalten:

| | RVA Max | RVA Min | RVA Fin | RVA Set | Peak Time | Pasting Temp |
|---|---|---|---|---|---|---|
| wt | 6112,0 | 2939 | 3820 | 881 | 4,14 | 70,15 |
| 432ES09 | 4252,0 | 3510 | 5327 | 1817 | 4,20 | 71,7 |
| 432ES22 | 2951,0 | 2833 | 4694 | 1861 | 5,64 | 75,25 |
| 432ES40 | 4577,0 | 3605 | 5039 | 1434 | 4,24 | 71,6 |

Tabelle 5: Bestimmung der Viskositätseigenschsften von Stärken, isoliert aus den Pflanzen 432ES09, 432ES33, 432ES40 und entsprechenden nicht transformierten Wildrtyp-Pflanzen (wt) mittels RVA Analyse. Die Werte sind in absoluten Einheiten (RVUs, Minuten bzw. °C) angegeben.

| | RVA Max | RVA Min | RVA Fin | RVA Set | Peak Time | Pasting Temp |
|---|---|---|---|---|---|---|
| wt | 100,0 | 100,0 | 100,0 | 100,0 | 100,0 | 100,0 |
| 432ES09 | 69,6 | 119,4 | 139,5 | 206,2 | 101,4 | 102,2 |
| 432ES22 | 48,3 | 96,4 | 122,9 | 211,2 | 136,2 | 107,3 |
| 432ES40 | 74,9 | 122,7 | 131,9 | 162,8 | 102,4 | 102,1 |

Tabelle 6: Dartsellung der in Tabelle 5 angegebenen Werte in relativen Einheiten in Prozent bezogen auf die Werte, erhalten für Stärke, isoliert aus entsprechenden nicht transformierten Wildtyp-Pflanzen (wt = 100%).

10. Bestimmung der Gelfestigkeit von Stärken, isoliert aus Pflanzen der Linie 432ES mittels eines Texture Analyzers

Die Analyse der Gelfestigkeit erfolgte nach der unter Allgemeine Methoden, Punkt 6 d) beschriebenen Methode. Es wurden forlgende Ergebnisse erhalten:

| | Kraft [g] |
|---|---|
| wt | 75,4 |
| 432ES09 | 182,2 |
| 432ES22 | 242,0 |
| 432ES40 | 172,0 |

Tabelle 7: Bestimmung der Gelfestigkeit von Stärken, isoliert aus den Pflanzen 432ES09, 432ES33, 432ES40 und entsprechenden nicht transformierten Wildrtyp-Pflanzen (wt) mittels Texture Analyzer. Dargestellt sind die absoluten Werte der Kraft in Gramm.

| | Kraft [%] |
|---|---|
| wt | 100,0 |
| 432ES09 | 241,7 |
| 432ES22 | 321,1 |
| 432ES40 | 228,2 |

Tabelle 8: Dartsellung der in Tabelle 7 angegebenen Werte in relativen Einheiten in Prozent bezogen auf die Werte, erhalten für Stärke, isoliert aus entsprechenden nicht transformierten Wildtyp-Pflanzen (wt = 100%).

11. Bestimmung der Korngröße von Stärken, isoliert aus Pflanzen der Linie 432ES Stärken von unabhängigen Pflanzen der Linie 432ES wurden wie unter Beispiel 7 beschrieben hergestellt und die Korngröße der betreffenden Stärkekörner anschließend nach der unter Allgemeine Methoden, Punkt 6 e) beschrieben Methode, ermittelt. Es wurden folgende Ergebnisse erhalten:

| | mittlere Korngröße [$\mu$m] |
|---|---|
| wt | 42.584 |
| 432ES09 | 25.116 |
| 432ES22 | 20.442 |
| 432ES40 | 26.119 |

Tabelle 9: Mittlere Größe von Stärkekörnern von Stärken, isoliert aus den Pflanzen 432ES09, 432ES33, 432ES40 und entsprechenden nicht transformierten Wildtyp-Pflanzen (wt).

12. Verdaubarkeit von gekochten Kartoffeln

Aus Knollen von unabhängigen Pflanzen der Linie 432ES wurden mit einem Korkbohrer (Durchmesser 4 mm) Zylinder ausgestanzt. Von diesen Zylindern wurden anschließend mit einem Skalpell etwa 1-2 mm dicke Scheiben abgeschnitten. Je 10 Scheiben eines Zylinders wurden in ein Reaktionsgefäß überführt, ca. 1 ml Wasser hinzu gegeben und für 5 Minuten in Wasser gekocht. Die gekochten Proben werden direkt nach dem Abkühlen auf Raumtemperatur mit 1 ml Pankreatinlösung für 4 Stunden bei 37°C inkubiert. Nach Beendigung des Verdaus mit Pankreatinlösung wurden die einzelnen Proben zur Darstellung des Anteils an unverdauter Stärke mittels Lugol'scher lösung angefärbt. Das Ergebnis dieses Versuches ist in Fig. 1 dargestellt. Die nach Anfärbung mit Lugol'scher Lösung auftretenden dunkeln Bereiche zeigen, dass in diesen Bereichen noch signifikante Mengen an Stärke vorliegen. Es handelt sich hierbei um RS. Helle bzw. nicht färbende Bereiche hingegen zeigen an, dass in diesen Bereichen keine Stärke mehr vorliegt, d.h. dass die Stärke zum größten Teil verdaut wurde.

Hersetllung der Pankreatinlösung:

3,75 g Pankreatin (aus Schweinepankreas von Merck, Produkt Nr. 1.07130.1000) wurden in 25 ml demineralisertem Wasser für 10 Minuten bei 37°C geschüttelt und anschließend für 10 Minuten bei 2000xg zentrifugiert. 21,06 ml des erhaltenen Überstandes wurden mit 3,85 ml demineralisiertem Wasser, 54,6 µl Amyloglukosidase (6000 U/ml, Sigma, Produkt Nr. A-3042,) und 100ml 0,1 M Na-Acetat-Puffer pH 5,2 + 4 mM $CaCl_2$ versetzt.

**Patentansprüche**

1. Verfahren zur Herstellung modifizierter Stärke in genetisch modifizierten Pflanzen, umfassend die folgenden Schritte

   a) Einführung eines fremden Nucleinsäuremoleküls codierend ein Protein mit der Aktivität einer Glutamin:Fructose-6-Phosphat Amidotransferase (GFAT) in eine Pflanzenzelle
   b) Regeneration einer Pflanze aus Pflanzenzellen erhalten nach Schritt a)
   c) gegebenenfalls Erzeugung von weiteren Pflanzen mit Hilfe der Pflanzen nach Schritt b).
   d) Extraktion von Stärke aus Pflanzen erhältlich nach Schritt b) oder c).

2. Modifizierte Stärke erhältlich nach einem Verfahren nach Anspruch 1.

3. Nahrungsmittel enthaltend eine Stärke nach Anspruch 2 oder eine Stärke, hergestellt nach einem Verfahren nach Anspruch 1.

4. Verfahren zur Herstellung eines Nahrungsmittels, worin Stärke nach Anspruch 2 oder Stärke, hergestellt nach einem Verfahren nach Anspruch 1 zu einem Nahrungsmittel hinzu gegeben wird.

5. Nahrungsmittel, enthaltend genetisch modifizierte Pflanzenzellen oder Teile von genetisch modifizierten Pflanzen, die ein fremdes Nucleinsäuremolekül, codierend ein Protein mit der Aktivität einer Glutamin:Fructose-6-Phosphat Amidotransferase (GFAT) aufweisen und die eine modifizierte Stärke nach Anspruch 2 oder hergestellt nach einem Verfahren nach Anspruch 1 aufweisen.

6. Verfahren zur Herstellung eines Nahrungsmittels nach Anspruch 5 umfassend die Verwendung von genetisch modifizierten Pflanzen oder Teile von genetisch modifizierten Pflanzen, die ein fremdes Nucleinsäuremolekül codierend ein Protein mit der Aktivität einer Glutamin:Fructose-6-Phosphat Amidotransferase aufweisen, zur Herstellung des Nahrungsmittels.

**7.** Nahrungsmittel erhältlich nach einem Verfahren nach Anspruch 6.

**8.** Verwendung einer genetisch modifizierten Pflanze die ein fremdes Nucleinsäuremolekül codierend ein Protein mit der Aktivität einer Glutamin:Fructose-6-Phosphat Amidotransferase aufweiset oder von modifizierter Stärke nach Anspruch 2 oder von modifizierter Stärke erhältlich nach einem Verfahren nach Anspruch 1 zur Herstellung eines Nahrungsmittels.

**9.** Verwendung eines Nucleinsäuremoleküs codierend ein Protein mit der Aktivität einer Glutamin:Fructose-6-Phosphat Amidotransferase (GFAT) zur Herstellung einer genetisch modifizierten Pflanze, die eine modifizierte Stärke synthetisiert.

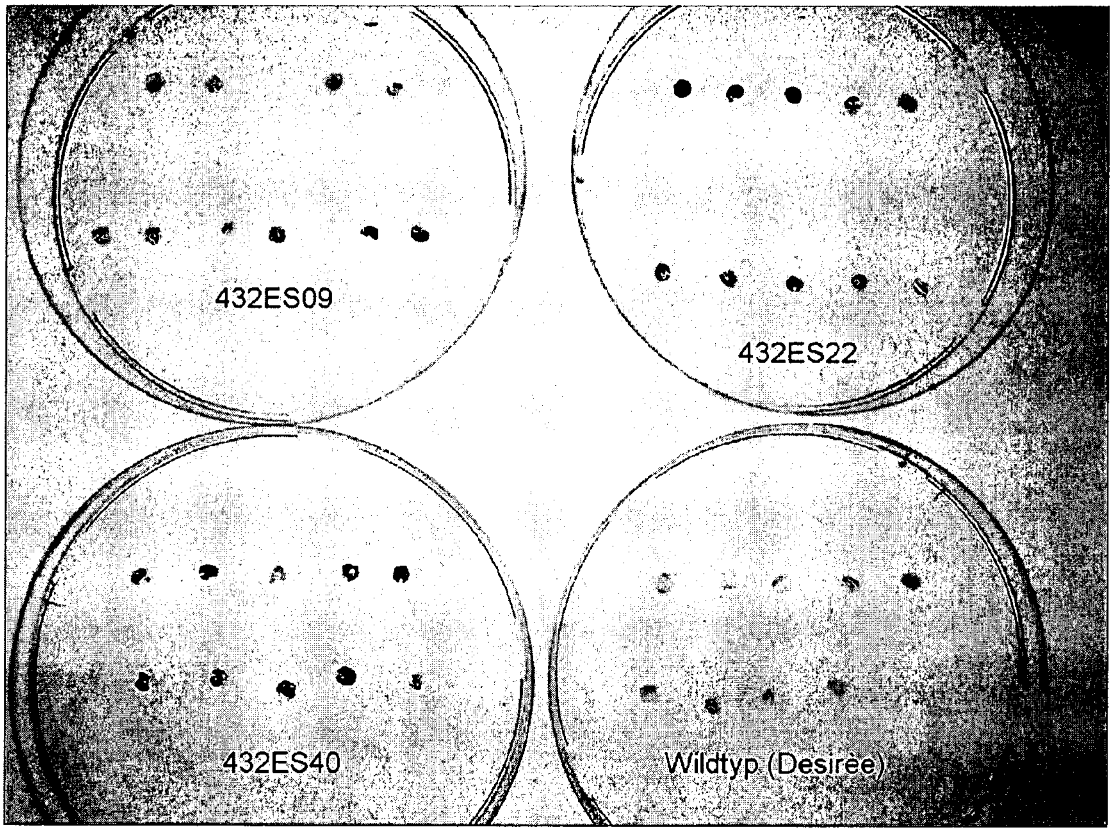


Fig. 1

Europäisches Patentamt

# EUROPÄISCHER RECHERCHENBERICHT

Nummer der Anmeldung
EP 07 09 0072

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 639 126 B1 (SEWALT VINCENT J H [US] ET AL) 28. Oktober 2003 (2003-10-28)<br>* Seite 1:25 - 8:67, 25:1 - 26:60, Ansprüche * | 1,4,6,8, 9 | INV.<br>C12N15/82<br>C08B30/04 |
| X | WO 00/11192 A (PIONEER HI BRED INT [US]) 2. März 2000 (2000-03-02)<br>* Seite 2:27 - 4:25, 29:20 - 38:23, 57:1 - 22, Ansprüche * | 1 | |
| E | WO 2007/039317 A (BAYER CROPSCIENCE GMBH [DE]; FROHBERG CLAUS [DE]; ESSIGMANN BERND [DE]) 12. April 2007 (2007-04-12)<br>* Seite 9:18 - 10:15, 13:32 - 17:25, 20:22 - 41:30, Beispiel 1-18,21, Ansprüche * | 1,4,6,8, 9 | |
| A | US 2004/003432 A1 (OBUKOWICZ MARK G [US]) 1. Januar 2004 (2004-01-01) | | |
| A | WO 02/37955 A (COMMW SCIENT IND RES ORG [AU]; MORELL MATTHEW KENNEDY [AU]; TOPPING DA) 16. Mai 2002 (2002-05-16) | | RECHERCHIERTE SACHGEBIETE (IPC) |
| A | US 5 977 454 A (MCNAUGHT KENNETH J [AU] ET AL) 2. November 1999 (1999-11-02) | | C12N<br>C08B |
| A | MILEWSKI S: "Glucosamine-6-phosphate synthase-the multi-facets enzyme"<br>BIOCHIMICA ET BIOPHYSICA ACTA. PROTEIN STRUCTURE AND MOLECULAR ENZYMOLOGY, ELSEVIER, AMSTERDAM,, NL,<br>Bd. 1597, Nr. 2,<br>3. Juni 2002 (2002-06-03), Seiten 173-192, XP004361024<br>ISSN: 0167-4838 | | |
| A | WO 2004/092391 A (ARKION LIFE SCIENCES LLC [US]) 28. Oktober 2004 (2004-10-28) | | |

-/--

Der vorliegende Recherchenbericht wurde für alle Patentansprüche erstellt

2

| Recherchenort | Abschlußdatum der Recherche | Prüfer |
|---|---|---|
| München | 4. September 2007 | PUONTI-KAERLAS, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
&: Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 07 09 0072

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 1 710 315 A (BAYER CROPSCIENCE GMBH [DE]) 11. Oktober 2006 (2006-10-11)<br>----- | | |
| A | DATABASE EMBL [Online]<br>28. Juni 2002 (2002-06-28), "Mus musculus glutamine fructose-6-phosphate transaminase 2, mRNA (cDNA clone MGC:18324 IMAGE:4167189), complete cds."<br>XP002449185<br>gefunden im EBI accession no. EMBL:BC031928<br>Database accession no. BC031928<br>& MAMMALIAN GENE COLLECTION (MGC) PROGRAM TEAM: "Generation and initial analysis of more than 15,000 full-length human and mouse cDNA sequences." PROCEEDINGS OF THE NATIONAL ACADEMY OF SCIENCES OF THE UNITED STATES OF AMERICA,<br>Bd. 99, Nr. 26,<br>24. Dezember 2002 (2002-12-24), Seiten 16899-16903, XP003015127<br>ISSN: 0027-8424<br>----- | | |
| A | TOPPING D L ET AL: "Resistant starch and health - Himalaya 292, a novel barley cultivar to deliver benefits to consumers"<br>STARKE - STARCH, WILEY-VCH VERLAG, WEINHEIM, DE,<br>Bd. 55, Nr. 12, 2003, Seiten 539-545, XP009086722<br>ISSN: 0038-9056<br>----- | | RECHERCHIERTE SACHGEBIETE (IPC) |

Der vorliegende Recherchenbericht wurde für alle Patentansprüche erstellt

2

| Recherchenort | Abschlußdatum der Recherche | Prüfer |
|---|---|---|
| München | 4. September 2007 | PUONTI-KAERLAS, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 07 09 0072

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-09-2007

| Im Recherchenbericht angeführtes Patentdokument | Datum der Veröffentlichung | Mitglied(er) der Patentfamilie | Datum der Veröffentlichung |
|---|---|---|---|
| US 6639126 B1 | 28-10-2003 | KEINE | |
| WO 0011192 A | 02-03-2000 | AU 6018399 A | 14-03-2000 |
| | | CA 2341078 A1 | 02-03-2000 |
| | | EP 1108040 A2 | 20-06-2001 |
| WO 2007039317 A | 12-04-2007 | KEINE | |
| US 2004003432 A1 | 01-01-2004 | KEINE | |
| WO 0237955 A | 16-05-2002 | AU 1480402 A | 21-05-2002 |
| | | AU 2006202440 A1 | 29-06-2006 |
| | | CA 2428259 A1 | 16-05-2002 |
| | | CN 1482856 A | 17-03-2004 |
| | | EP 1331845 A1 | 06-08-2003 |
| | | JP 2004512427 T | 22-04-2004 |
| | | US 2004199942 A1 | 07-10-2004 |
| US 5977454 A | 02-11-1999 | AT 211610 T | 15-01-2002 |
| | | AT 224135 T | 15-10-2002 |
| | | WO 9403049 A1 | 17-02-1994 |
| | | CA 2118038 A1 | 17-02-1994 |
| | | DE 69331439 D1 | 14-02-2002 |
| | | DE 69331439 T2 | 05-09-2002 |
| | | DE 69332316 D1 | 24-10-2002 |
| | | DE 69332316 T2 | 18-06-2003 |
| | | DK 652701 T3 | 29-04-2002 |
| | | DK 0885556 T3 | 27-01-2003 |
| | | EP 0652701 A1 | 17-05-1995 |
| | | ES 2171413 T3 | 16-09-2002 |
| | | ES 2184197 T3 | 01-04-2003 |
| | | JP 8503123 T | 09-04-1996 |
| | | JP 3628690 B2 | 16-03-2005 |
| | | JP 2004156040 A | 03-06-2004 |
| | | NZ 254014 A | 24-11-1997 |
| | | PT 652701 T | 28-06-2002 |
| | | PT 885556 T | 31-12-2002 |
| | | US 5714600 A | 03-02-1998 |
| | | US 6409840 B1 | 25-06-2002 |
| WO 2004092391 A | 28-10-2004 | KEINE | |
| EP 1710315 A | 11-10-2006 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 564893 A1
- EP 688872 A1
- EP 846704 A1
- US 5051271 A
- US 5714600 A
- WO 20040199942 A
- WO 0011192 A
- WO 0173087 A
- WO 9307279 A
- WO 04003175 A
- EP 120516 A
- US 5565347 A
- WO 9506128 A
- EP 0513849 A
- EP 0465875 A
- EP 0292435 A
- WO 1782002 A


### In der Beschreibung aufgeführte Nicht-Patentliteratur


- **HIZUKURI ; TAKAGI.** *Carbohydr. Res.,* 1984, vol. 134, 1-10
- **TAKEDA et al.** *Carbohydr. Res.,* 1984, vol. 132, 83-92
- **FAISANT et al.** *Sciences des Aliments,* 1995, vol. 15, 83-89
- **EVANS ; THOMPSON.** *Cereal Chemistry,* 2004, vol. 81 (1), 31-37
- **ENGLYST et al.** *European Journal of Clinical Nutrition,* 1992, vol. 46 (2), S33-S50
- **SAMBROK et al.** Molecular Cloning, A Laboratory Manual. Cold Spring Harbour Laboratory Press, 2001
- **AUSUBEL et al.** Short Protocols in Molecular Biology. John Wiley & Sons, 2002
- **STAVOLONE et al.** *Plant Mol. Biol.,* 2003, vol. 53, 703-713
- **ROCHA-SOSA et al.** *EMBO J.,* 1989, vol. 8, 23-29
- **MONTGOMERY et al.** *Plant Cell,* 1993, vol. 5, 1049-1062
- **METHA et al.** *Nature Biotechnol.,* 2002, vol. 20 (6), 613-618
- **MOON ; CALLAHAN.** *J. Experimental Botany,* 2004, vol. 55 (402), 1519-1528
- **STOCKHAUS et al.** *Proc. Natl. Acad. Sci. USA,* 1987, vol. 84, 7943-7947
- **STOCKHAUS et al.** *EMBO J.,* 1989, vol. 8, 2445-2451
- **PEDERSEN et al.** *Cell,* 1982, vol. 29, 1015-1026
- **QUATROCCIO et al.** *Plant Mol. Biol.,* 1990, vol. 15, 81-93
- **LEISY et al.** *Plant Mol. Biol.,* 1990, vol. 14, 41-50
- **ZHENG et al.** *Plant J.,* 1993, vol. 4, 357-366
- **YOSHIHARA et al.** *FEBS Lett.,* vol. 383, 213-218
- **NAKASE et al.** *Gene,* 1996, vol. 170 (2), 223-226
- **QU ; TAKAIWA.** *Plant Biotechnology Journal,* 2004, vol. 2 (2), 113-125
- **WERR et al.** *EMBO J.,* 1985, vol. 4, 1373-1380
- **FIEDLER et al.** *Plant Mol. Biol.,* 1993, vol. 22, 669-679
- **BÄUMLEIN et al.** *Mol. Gen. Genet.,* 1991, vol. 225, 459-467
- **MITRA et al.** *Biochem. Biophys Res Commun,* 1994, vol. 204 (1), 187-194
- **MITRA ; HIGGINS.** *Plant Mol Biol,* 1994, vol. 26 (1), 85-93
- **VAN ETTEN et al.** *Arch Virol,* 2002, vol. 147, 1479-1516
- **GIELEN et al.** *EMBO J.,* 1989, vol. 8, 23-29
- **CALLIS et al.** *Genes Devel.,* 1987, vol. 1, 1183-1200
- **LUEHRSEN ; WALBOT.** *Mol. Gen. Genet.,* 1991, vol. 225, 81-93
- **RETHMEIER et al.** *Plant Journal,* 1997, vol. 12 (4), 895-899
- **ROSE ; BELIAKOFF.** *Plant Physiol.,* 2000, vol. 122 (2), 535-542
- **VASIL et al.** *Plant Physiol.,* 1989, vol. 91, 1575-1579
- **XU et al.** *Science in China Series C,* 2003, vol. 46 (6), 561-569
- **MILEWSKI.** *Biochimica et Biophysica Acta,* 2002, vol. 1597, 173-193
- **HU et al.** *J. Biol. Chem.,* 2004, vol. 279 (29), 29988-29993

- **HUBER ; HARDIN.** *Current Opinion in Plant Biotechnology,* 2004, vol. 7, 318-322
- **KORNFELD.** *J. Biol. Chem.,* 1967, vol. 242 (13), 3135-3141
- **BROSCHAT et al.** *J. Biol. Cehm.,* 2002, vol. 277 (17), 14764-14770
- **DENG et al.** *Metabolic Engeneering,* 2005, vol. 7, 201-214
- **DENG et al.** *Metabolic Engineering,* 2005, vol. 7, 201-214
- **DUTKA-MALEN.** *Biochemie,* 1988, vol. 70 (2), 287-290
- **WATZELE et al.** *J. Biol. Chem.,* 1989, vol. 264, 8753-8758
- **KATO et al.** *Insect. Biol.,* 2002, vol. 11 (3), 207, 216
- **OKI et al.** *Genomics,* 1999, vol. 57 (2), 227-34
- **SAMBROCK et al.** Molecular Cloning, A Laboratory Manual. Cold Spring Harbor Laboratory Press, 1989
- **SAMBROOK et al.** Molecular Cloning, A Laboratory Manual. Cold Spring Harbor Laboratory Press, 1989
- **MAYER et al.** *Plant Physiol.,* 1968, vol. 43, 1097-1107
- **THOMPSON et al.** *Nucleic Acids Research,* 1994, vol. 22, 4673-4680
- **HOEKEMA.** The Binary Plant Vector System Offsetdrukkerij Kanters B.V. Alblasserdam. 1985
- **FRALEY et al.** *Crit. Rev. Plant Sci.,* 1985, vol. 4, 1-46
- **AN et al.** *EMBO J.,* 1985, vol. 4, 277-287
- **ROCHA-SOSA et al.** *EMBO J.,* 1989, vol. 8, 29-33
- **CHAN et al.** *Plant Mol. Biol.,* 1993, vol. 22, 491-506
- **HIEI et al.** *Plant J.,* 1994, vol. 6, 271-282
- **DENG et al.** *Science in China,* 1990, vol. 33, 28-34
- **WILMINK et al.** *Plant Cell Reports,* 1992, vol. 11, 76-80
- **MAY et al.** *Bio/Technology,* 1995, vol. 13, 486-492
- **CONNER ; DOMISSE.** *Int. J. Plant Sci.,* 1992, vol. 153, 550-555
- **RITCHIE et al.** *Transgenic Res.,* 1993, vol. 2, 252-265
- **WAN ; LEMAUX.** *Plant Physiol.,* 1994, vol. 104, 37-48
- **VASIL et al.** *Bio/Technology,* 1993, vol. 11, 1553-1558
- **RITALA et al.** *Plant Mol. Biol.,* 1994, vol. 24, 317-325
- **SPENCER et al.** *Theor. Appl. Genet.,* 1990, vol. 79, 625-631
- **FROMM et al.** *Biotechnology,* 1990, vol. 8, 833-844
- **GORDON-KAMM et al.** *Plant Cell,* 1990, vol. 2, 603-618
- **KOZIEL et al.** *Biotechnology,* 1993, vol. 11, 194-200
- **MOROC et al.** *Theor. Appl. Genet.,* 1990, vol. 80, 721-726
- **RICHARDS et al.** *Plant Cell Reporters,* 2001, vol. 20, 48-54
- **KRENS et al.** *Nature,* 1982, vol. 296, 72-74
- **NEHRA et al.** *Plant J.,* 1994, vol. 5, 285-297
- **BECKER et al.** *Plant Journal,* 1994, vol. 5, 299-307
- **NAM et al.** *The Plant Cell,* 1989, vol. 1, 699-705
- **LEISTER ; DEAN.** *The Plant Journal,* 1993, vol. 4 (4), 745-750
- **CASTIGLIONI et al.** *Genetics,* 1998, vol. 149, 2039-2056
- **MEKSEM et al.** *Molecular Genetics and Genomics,* 2001, vol. 265, 207-214
- **MEYER et al.** *Molecular and General Genetics,* 1998, vol. 259, 150-160
- **KONIECZNY ; AUSUBEL.** *The Plant Journal,* 1993, vol. 4, 403-410
- **JARVIS et al.** *Plant Molecular Biology,* 1994, vol. 24, 685-687
- **BACHEM et al.** *The Plant Journal,* 1996, vol. 9 (5), 745-753
- Plant Cell Culture Protocols. Humana Press, 1999
- **STARCH ; WHISTLER ; BEMILLER ; PASCHALL.** Chemistry and Technology. Academic Press Inc. London Ltd, 1994
- **WATSON.** Mais und Sorghum Stärken: Herstellung. 412-468
- **CORBISHLEY ; MILLER.** Tapioca, Arrowroot und Sago Stärken: Herstellung. 469-479
- **MITCH.** Kartoffelstärke: Herstellung und Verwendungen. 479-490
- **KNIGHT ; OSON.** Weizenstärke: Herstellung, Modifizierung und Verwendungen. 491-506
- **ROHMER ; KLEM.** Reisstärke: Herstellung und Verwendungen. 507-528
- **ECKHOFF et al.** *Cereal Chem.,* 1996, vol. 73, 54-57
- **ENGLYST et al.** *Europ. J. of Clinical Nutrition,* 1992, vol. 46 (2), 33-50
- **LIEPKE et al.** *Eur. J. Biochem.,* 2002, vol. 269, 712-718
- **ADOLFO BUCIO GALINDO.** *Proefschrift, Wageningen Universiteit,* 2004, ISBN 90-5808-943-6
- **MORGAN ; ELSON.** *Biochem J.,* 1934, vol. 28 (3), 988-995
- **ELSON ; MORGAN.** *J Biochem.,* 1933, vol. 27, 1824
- **REISSIG et al.** *Biol. Chem.,* 1955, vol. 217, 959-966

- **MUCKENSCHNABEL et al.** *Cancer Letters,* 1998, vol. 131, 13-20
- **RACHEL et al.** *J. Bacteriol.,* 1996, vol. 178 (8), 2320-2327
- **HOVENKAMP-HERMELINK et al.** beschriebenen Methode. *Potato Research,* 1988, vol. 31, 241-246
- **ENGLYST et al.** *Reagents, Apparatus, Spectrophotometer,* S35-S36
- **ABSATZ.** *Measurement of free glucose (FG,* S36-S37
- **ABSATZ.** *Measurement of RDS and SDS,* S38
- **ZHANG et al.** *Biomacromolecules,* 2006, vol. 7, 3252-3258
- *Methods. Enzymatic Starch Hydrolysis,* 3253
- **HÖFGEN ; WILLMITZER.** *Plant Science,* 1990, vol. 66, 221-230
- **BECKER.** *Nucleic Acids Res.,* 1990, vol. 18, 203

SEQUENCE LISTING

<110> Bayer CropScience AG

<120> Verfahren zur Herstellung einer resistenten Stärke

<130> BCS 07-5004 EP

<160> 5

<170> PatentIn version 3.3

<210> 1
<211> 1830
<212> DNA
<213> Escherichia coli

<220>
<221> CDS
<222> (1)..(1827)

<300>
<308> U00096.2
<309> 2005-09-08
<313> (3909862)..(3911691)

<400> 1

```
atg tgt gga att gtt ggc gcg atc gcg caa cgt gat gta gca gaa atc      48
Met Cys Gly Ile Val Gly Ala Ile Ala Gln Arg Asp Val Ala Glu Ile
1               5                   10                  15

ctt ctt gaa ggt tta cgt cgt ctg gaa tac cgc gga tat gac tct gcc      96
Leu Leu Glu Gly Leu Arg Arg Leu Glu Tyr Arg Gly Tyr Asp Ser Ala
            20                  25                  30

ggt ctg gcc gtt gtt gat gca gaa ggt cat atg acc cgc ctg cgt cgc     144
Gly Leu Ala Val Val Asp Ala Glu Gly His Met Thr Arg Leu Arg Arg
        35                  40                  45

ctc ggt aaa gtc cag atg ctg gca cag gca gcg gaa gaa cat cct ctg     192
Leu Gly Lys Val Gln Met Leu Ala Gln Ala Ala Glu Glu His Pro Leu
    50                  55                  60

cat ggc ggc act ggt att gct cac act cgc tgg gcg acc cac ggt gaa     240
His Gly Gly Thr Gly Ile Ala His Thr Arg Trp Ala Thr His Gly Glu
65                  70                  75                  80
```

```
cct tca gaa gtg aat gcg cat ccg cat gtt tct gaa cac att gtg gtg       288
Pro Ser Glu Val Asn Ala His Pro His Val Ser Glu His Ile Val Val
                85                  90                  95

gtg cat aac ggc atc atc gaa aac cat gaa ccg ctg cgt gaa gag cta       336
Val His Asn Gly Ile Ile Glu Asn His Glu Pro Leu Arg Glu Glu Leu
            100                 105                 110

aaa gcg cgt ggc tat acc ttc gtt tct gaa acc gac acc gaa gtg att       384
Lys Ala Arg Gly Tyr Thr Phe Val Ser Glu Thr Asp Thr Glu Val Ile
        115                 120                 125

gcc cat ctg gtg aac tgg gag ctg aaa caa ggc ggg act ctg cgt gag       432
Ala His Leu Val Asn Trp Glu Leu Lys Gln Gly Gly Thr Leu Arg Glu
    130                 135                 140

gcc gtt ctg cgt gct atc ccg cag ctg cgt ggt gcg tac ggt aca gtg       480
Ala Val Leu Arg Ala Ile Pro Gln Leu Arg Gly Ala Tyr Gly Thr Val
145                 150                 155                 160

atc atg gac tcc cgt cac ccg gat acc ctg ctg gcg gca cgt tct ggt       528
Ile Met Asp Ser Arg His Pro Asp Thr Leu Leu Ala Ala Arg Ser Gly
                165                 170                 175

agt ccg ctg gtg att ggc ctg ggg atg ggc gaa aac ttt atc gct tct       576
Ser Pro Leu Val Ile Gly Leu Gly Met Gly Glu Asn Phe Ile Ala Ser
            180                 185                 190

gac cag ctg gcg ctg ttg ccg gtg acc cgt cgc ttt atc ttc ctt gaa       624
Asp Gln Leu Ala Leu Leu Pro Val Thr Arg Arg Phe Ile Phe Leu Glu
        195                 200                 205

gag ggc gat att gcg gaa atc act cgc cgt tcg gta aac atc ttc gat       672
Glu Gly Asp Ile Ala Glu Ile Thr Arg Arg Ser Val Asn Ile Phe Asp
    210                 215                 220

aaa act ggc gcg gaa gta aaa cgt cag gat atc gaa tcc aat ctg caa       720
Lys Thr Gly Ala Glu Val Lys Arg Gln Asp Ile Glu Ser Asn Leu Gln
225                 230                 235                 240

tat gac gcg ggc gat aaa ggc att tac cgt cac tac atg cag aaa gag       768
Tyr Asp Ala Gly Asp Lys Gly Ile Tyr Arg His Tyr Met Gln Lys Glu
                245                 250                 255

atc tac gaa cag ccg aac gcg atc aaa aac acc ctt acc gga cgc atc       816
Ile Tyr Glu Gln Pro Asn Ala Ile Lys Asn Thr Leu Thr Gly Arg Ile
            260                 265                 270
```

```
agc cac ggt cag gtt gat tta agc gag ctg gga ccg aac gcc gac gaa      864
Ser His Gly Gln Val Asp Leu Ser Glu Leu Gly Pro Asn Ala Asp Glu
        275                 280                 285

ctg ctg tcg aag gtt gag cat att cag atc ctc gcc tgt ggt act tct      912
Leu Leu Ser Lys Val Glu His Ile Gln Ile Leu Ala Cys Gly Thr Ser
    290                 295                 300

tat aac tcc ggt atg gtt tcc cgc tac tgg ttt gaa tcg cta gca ggt      960
Tyr Asn Ser Gly Met Val Ser Arg Tyr Trp Phe Glu Ser Leu Ala Gly
305                 310                 315                 320

att ccg tgc gac gtc gaa atc gcc tct gaa ttc cgc tat cgc aaa tct     1008
Ile Pro Cys Asp Val Glu Ile Ala Ser Glu Phe Arg Tyr Arg Lys Ser
                325                 330                 335

gcc gtg cgt cgt aac agc ctg atg atc acc ttg tca cag tct ggc gaa     1056
Ala Val Arg Arg Asn Ser Leu Met Ile Thr Leu Ser Gln Ser Gly Glu
            340                 345                 350

acc gcg gat acc ctg gct ggc ctg cgt ctg tcg aaa gag ctg ggt tac     1104
Thr Ala Asp Thr Leu Ala Gly Leu Arg Leu Ser Lys Glu Leu Gly Tyr
        355                 360                 365

ctt ggt tca ctg gca atc tgt aac gtt ccg ggt tct tct ctg gtg cgc     1152
Leu Gly Ser Leu Ala Ile Cys Asn Val Pro Gly Ser Ser Leu Val Arg
    370                 375                 380

gaa tcc gat ctg gcg cta atg acc aac gcg ggt aca gaa atc ggc gtg     1200
Glu Ser Asp Leu Ala Leu Met Thr Asn Ala Gly Thr Glu Ile Gly Val
385                 390                 395                 400

gca tcc act aaa gca ttc acc act cag tta act gtg ctg ttg atg ctg     1248
Ala Ser Thr Lys Ala Phe Thr Thr Gln Leu Thr Val Leu Leu Met Leu
                405                 410                 415

gtg gcg aag ctg tct cgc ctg aaa ggt ctg gat gcc tcc att gaa cat     1296
Val Ala Lys Leu Ser Arg Leu Lys Gly Leu Asp Ala Ser Ile Glu His
            420                 425                 430

gac atc gtg cat ggt ctg cag gcg ctg ccg agc cgt att gag cag atg     1344
Asp Ile Val His Gly Leu Gln Ala Leu Pro Ser Arg Ile Glu Gln Met
        435                 440                 445

ctg tct cag gac aaa cgc att gaa gcg ctg gca gaa gat ttc tct gac     1392
Leu Ser Gln Asp Lys Arg Ile Glu Ala Leu Ala Glu Asp Phe Ser Asp
    450                 455                 460
```

```
aaa cat cac gcg ctg ttc ctg ggc cgt ggc gat cag tac cca atc gcg      1440
Lys His His Ala Leu Phe Leu Gly Arg Gly Asp Gln Tyr Pro Ile Ala
465                 470                 475                 480

ctg gaa ggc gca ttg aag ttg aaa gag atc tct tac att cac gct gaa      1488
Leu Glu Gly Ala Leu Lys Leu Lys Glu Ile Ser Tyr Ile His Ala Glu
                485                 490                 495

gcc tac gct gct ggc gaa ctg aaa cac ggt ccg ctg gcg cta att gat      1536
Ala Tyr Ala Ala Gly Glu Leu Lys His Gly Pro Leu Ala Leu Ile Asp
            500                 505                 510

gcc gat atg ccg gtt att gtt gtt gca ccg aac aac gaa ttg ctg gaa      1584
Ala Asp Met Pro Val Ile Val Val Ala Pro Asn Asn Glu Leu Leu Glu
        515                 520                 525

aaa ctg aaa tcc aac att gaa gaa gtt cgc gcg cgt ggc ggt cag ttg      1632
Lys Leu Lys Ser Asn Ile Glu Glu Val Arg Ala Arg Gly Gly Gln Leu
    530                 535                 540

tat gtc ttc gcc gat cag gat gcg ggt ttt gta agt agc gat aac atg      1680
Tyr Val Phe Ala Asp Gln Asp Ala Gly Phe Val Ser Ser Asp Asn Met
545                 550                 555                 560

cac atc atc gag atg ccg cat gtg gaa gag gtg att gca ccg atc ttc      1728
His Ile Ile Glu Met Pro His Val Glu Glu Val Ile Ala Pro Ile Phe
                565                 570                 575

tac acc gtt ccg ctg cag ctg ctg gct tac cat gtc gcg ctg atc aaa      1776
Tyr Thr Val Pro Leu Gln Leu Leu Ala Tyr His Val Ala Leu Ile Lys
            580                 585                 590

ggc acc gac gtt gac cag ccg cgt aac ctg gca aaa tcg gtt acg gtt      1824
Gly Thr Asp Val Asp Gln Pro Arg Asn Leu Ala Lys Ser Val Thr Val
        595                 600                 605

gag taa                                                              1830
Glu


<210>  2
<211>  609
<212>  PRT
<213>  Escherichia coli

<400>  2
```

```
Met Cys Gly Ile Val Gly Ala Ile Ala Gln Arg Asp Val Ala Glu Ile
1               5                   10                  15

Leu Leu Glu Gly Leu Arg Arg Leu Glu Tyr Arg Gly Tyr Asp Ser Ala
            20                  25                  30

Gly Leu Ala Val Val Asp Ala Glu Gly His Met Thr Arg Leu Arg Arg
        35                  40                  45

Leu Gly Lys Val Gln Met Leu Ala Gln Ala Ala Glu Glu His Pro Leu
    50                  55                  60

His Gly Gly Thr Gly Ile Ala His Thr Arg Trp Ala Thr His Gly Glu
65                  70                  75                  80

Pro Ser Glu Val Asn Ala His Pro His Val Ser Glu His Ile Val Val
                85                  90                  95

Val His Asn Gly Ile Ile Glu Asn His Glu Pro Leu Arg Glu Glu Leu
            100                 105                 110

Lys Ala Arg Gly Tyr Thr Phe Val Ser Glu Thr Asp Thr Glu Val Ile
        115                 120                 125

Ala His Leu Val Asn Trp Glu Leu Lys Gln Gly Gly Thr Leu Arg Glu
    130                 135                 140

Ala Val Leu Arg Ala Ile Pro Gln Leu Arg Gly Ala Tyr Gly Thr Val
145                 150                 155                 160

Ile Met Asp Ser Arg His Pro Asp Thr Leu Leu Ala Ala Arg Ser Gly
                165                 170                 175

Ser Pro Leu Val Ile Gly Leu Gly Met Gly Glu Asn Phe Ile Ala Ser
            180                 185                 190
```

```
Asp Gln Leu Ala Leu Leu Pro Val Thr Arg Arg Phe Ile Phe Leu Glu
        195                 200                 205

Glu Gly Asp Ile Ala Glu Ile Thr Arg Arg Ser Val Asn Ile Phe Asp
    210                 215                 220

Lys Thr Gly Ala Glu Val Lys Arg Gln Asp Ile Glu Ser Asn Leu Gln
225                 230                 235                 240

Tyr Asp Ala Gly Asp Lys Gly Ile Tyr Arg His Tyr Met Gln Lys Glu
                245                 250                 255

Ile Tyr Glu Gln Pro Asn Ala Ile Lys Asn Thr Leu Thr Gly Arg Ile
            260                 265                 270

Ser His Gly Gln Val Asp Leu Ser Glu Leu Gly Pro Asn Ala Asp Glu
        275                 280                 285

Leu Leu Ser Lys Val Glu His Ile Gln Ile Leu Ala Cys Gly Thr Ser
    290                 295                 300

Tyr Asn Ser Gly Met Val Ser Arg Tyr Trp Phe Glu Ser Leu Ala Gly
305                 310                 315                 320

Ile Pro Cys Asp Val Glu Ile Ala Ser Glu Phe Arg Tyr Arg Lys Ser
                325                 330                 335

Ala Val Arg Arg Asn Ser Leu Met Ile Thr Leu Ser Gln Ser Gly Glu
            340                 345                 350

Thr Ala Asp Thr Leu Ala Gly Leu Arg Leu Ser Lys Glu Leu Gly Tyr
        355                 360                 365

Leu Gly Ser Leu Ala Ile Cys Asn Val Pro Gly Ser Ser Leu Val Arg
    370                 375                 380
```

```
Glu Ser Asp Leu Ala Leu Met Thr Asn Ala Gly Thr Glu Ile Gly Val
385                 390                 395                 400

Ala Ser Thr Lys Ala Phe Thr Thr Gln Leu Thr Val Leu Leu Met Leu
                405                 410                 415

Val Ala Lys Leu Ser Arg Leu Lys Gly Leu Asp Ala Ser Ile Glu His
            420                 425                 430

Asp Ile Val His Gly Leu Gln Ala Leu Pro Ser Arg Ile Glu Gln Met
        435                 440                 445

Leu Ser Gln Asp Lys Arg Ile Glu Ala Leu Ala Glu Asp Phe Ser Asp
    450                 455                 460

Lys His His Ala Leu Phe Leu Gly Arg Gly Asp Gln Tyr Pro Ile Ala
465                 470                 475                 480

Leu Glu Gly Ala Leu Lys Leu Lys Glu Ile Ser Tyr Ile His Ala Glu
                485                 490                 495

Ala Tyr Ala Ala Gly Glu Leu Lys His Gly Pro Leu Ala Leu Ile Asp
            500                 505                 510

Ala Asp Met Pro Val Ile Val Val Ala Pro Asn Asn Glu Leu Leu Glu
        515                 520                 525

Lys Leu Lys Ser Asn Ile Glu Glu Val Arg Ala Arg Gly Gly Gln Leu
    530                 535                 540

Tyr Val Phe Ala Asp Gln Asp Ala Gly Phe Val Ser Ser Asp Asn Met
545                 550                 555                 560

His Ile Ile Glu Met Pro His Val Glu Glu Val Ile Ala Pro Ile Phe
                565                 570                 575
```

Tyr Thr Val Pro Leu Gln Leu Leu Ala Tyr His Val Ala Leu Ile Lys
580 585 590

Gly Thr Asp Val Asp Gln Pro Arg Asn Leu Ala Lys Ser Val Thr Val
595 600 605

Glu

<210> 3
<211> 1830
<212> DNA
<213> Artificial Sequence

<220>
<223> Synthetic sequence encoding an Escherichia coli protein having the activity of a GFAT

<400> 3
atgtgcggaa ttgttggtgc tatcgcccaa agagacgttg ctgagatttt gttagagggt 60

ctgcgaaggc tagagtatag aggatatgac tccgctggtc tggctgtcgt tgatgctgag 120

ggtcatatga caaggctaag aaggttagga aaggttcaga tgcttgctca ggcagctgag 180

gaacatccat tgcatggagg tactggtatt gcacatacca ggtgggctac tcatggggag 240

ccatcagaag ttaatgctca tccacatgtg agtgagcata tcgttgtagt tcacaatggg 300

ataattgaaa accacgaacc attgagggaa gagttaaagg caagaggata tacttttgtg 360

agtgagactg acactgaggt tattgcacat ttagtgaact gggaactcaa acagggggc 420

acattgcgtg aggctgtgtt aagagctatt cctcaactta gaggtgcata cggtactgtt 480

attatggatt caagacaccc agatactctc cttgcagcta gatcaggtag tcccttggtc 540

ataggacttg gaatgggtga aaattttatc gctagcgacc aattggcctt attgccagtt 600

acaagacgat ttattttcct tgaagagggc gatattgctg agattactag aaggtctgtg 660

aacatctttg ataagactgg cgctgaggtt aaacgtcagg atatcgagtc taaccttcaa 720

tacgatgctg gtgataaagg aatttacagg cattatatgc aaaaggaaat ttatgaacaa 780

```
ccaaatgcta tcaaaaacac acttactggc cgtatttctc atggacaggt cgatttaagc    840

gagcttggtc ctaatgcaga cgaactgcta tcaaaagttg agcacataca gatactggca    900

tgcggaacta gttataattc aggaatggtc tctagatact ggttcgaaag cttggcaggt    960

ataccttgtg atgtagagat cgcttctgag tttaggtata gaaagtctgc tgtgcgtaga   1020

aattcattaa tgattacatt atctcaatcc ggagaaacag cagatacact ggctggattg   1080

aggctttcta aggaactcgg atatctgggt tcacttgcta tttgtaatgt accaggttcc   1140

tcattggttc gtgaatcaga tctagcactt atgacaaatg caggaactga aataggtgtg   1200

gcaagtacca aggctttcac aacccaactg accgtacttt taatgttggt agcaaaactc   1260

agtcgattaa aggggctaga tgcatctatc gaacatgata ttgttcacgg gcttcaagct   1320

ctcccttcaa gaattgaaca aatgctttca caagataaga gaatagaggc attggctgaa   1380

gatttttccg acaaacatca cgcattgttt cttggacgtg gcgatcaata tccaattgca   1440

ttggaaggag ctttgaagtt gaaagaaata agttacattc acgcagaagc atatgcagct   1500

ggagaactca agcatggtcc tttggcactc atcgacgctg acatgcccgt gatcgtagtg   1560

gctcctaata acgaactgct cgaaaagctt aaatcaaata tcgaagaggt tcgagctaga   1620

ggaggtcagc tttacgtttt cgctgaacaa gatgctggat tcgtgtcaag cgataatatg   1680

catataattg aaatgcctca cgttgaagaa gtgattgcac ctatatttta tacagtccca   1740

ttgcaacttc tagcttacca tgttgcactt attaaaggaa ctgatgttga tcagcctaga   1800

aacctagcaa aatctgtaac agtcgaataa                                    1830
```

<210> 4
<211> 2049
<212> DNA
<213> Mus musculus

<220>
<221> CDS
<222> (1)..(2046)

<300>

<308> BC031928.1
<309> 2003-10-07
<313> (51)..(299)

<400> 4
```
atg tgc gga atc ttt gcc tac atg aat tac aga gtt ccc aag aca agg        48
Met Cys Gly Ile Phe Ala Tyr Met Asn Tyr Arg Val Pro Lys Thr Arg
1               5                   10                  15

aaa gag att ttc gaa acc ctt atc agg ggt ctg cag cgg ctg gag tac        96
Lys Glu Ile Phe Glu Thr Leu Ile Arg Gly Leu Gln Arg Leu Glu Tyr
            20                  25                  30

cgg ggc tat gac tct gcg ggg gtt gcc att gat ggg aat aac cac gaa       144
Arg Gly Tyr Asp Ser Ala Gly Val Ala Ile Asp Gly Asn Asn His Glu
        35                  40                  45

gtc aaa gaa aga cac atc cat ctt gtg aag aaa agg ggg aaa gta aag       192
Val Lys Glu Arg His Ile His Leu Val Lys Lys Arg Gly Lys Val Lys
    50                  55                  60

gct ctg gat gaa gaa ctt tac aag caa gat agc atg gac ttg aag gtg       240
Ala Leu Asp Glu Glu Leu Tyr Lys Gln Asp Ser Met Asp Leu Lys Val
65                  70                  75                  80

gag ttt gag aca cac ttc ggc att gcc cac aca cgt tgg gcc acc cac       288
Glu Phe Glu Thr His Phe Gly Ile Ala His Thr Arg Trp Ala Thr His
                85                  90                  95

ggg gtt ccc aat gct gtc aac agt cac ccg cag cgt tcg gac aaa gac       336
Gly Val Pro Asn Ala Val Asn Ser His Pro Gln Arg Ser Asp Lys Asp
            100                 105                 110

aat gaa ttt gtt gtc atc cac aac ggg atc atc act aat tac aag gat       384
Asn Glu Phe Val Val Ile His Asn Gly Ile Ile Thr Asn Tyr Lys Asp
        115                 120                 125

cta agg aag ttt ctg gaa agc aaa ggc tac gag ttt gag tca gaa aca       432
Leu Arg Lys Phe Leu Glu Ser Lys Gly Tyr Glu Phe Glu Ser Glu Thr
    130                 135                 140

gac acg gag acc atc gcc aag ctg att aaa tat gta ttt gac aac aga       480
Asp Thr Glu Thr Ile Ala Lys Leu Ile Lys Tyr Val Phe Asp Asn Arg
145                 150                 155                 160

gag act gag gac ata acg ttt tcc aca ttg gtc gaa aga gtc att cag       528
Glu Thr Glu Asp Ile Thr Phe Ser Thr Leu Val Glu Arg Val Ile Gln
                165                 170                 175
```

```
cag ttg gaa ggc gcc ttt gca ctg gtt ttc aag agt att cac tac ccg      576
Gln Leu Glu Gly Ala Phe Ala Leu Val Phe Lys Ser Ile His Tyr Pro
            180                 185                 190

gga gaa gct gtc gcc acg agg aga ggc agc ccc ttg ctc atc ggg gta      624
Gly Glu Ala Val Ala Thr Arg Arg Gly Ser Pro Leu Leu Ile Gly Val
        195                 200                 205

cga agc aaa tac aaa ctc tcc aca gag cag atc ccc gtc tta tat ccg      672
Arg Ser Lys Tyr Lys Leu Ser Thr Glu Gln Ile Pro Val Leu Tyr Pro
    210                 215                 220

aca tgc aat atc gag aat gtg aag aat atc tgc aag act agg atg aag      720
Thr Cys Asn Ile Glu Asn Val Lys Asn Ile Cys Lys Thr Arg Met Lys
225                 230                 235                 240

aga ctg gac agc tcc acc tgc ctg cac gct gtg ggc gat aaa gct gtg      768
Arg Leu Asp Ser Ser Thr Cys Leu His Ala Val Gly Asp Lys Ala Val
                245                 250                 255

gaa ttc ttc ttt gct tct gat gca agt gcc atc ata gaa cac acc aac      816
Glu Phe Phe Phe Ala Ser Asp Ala Ser Ala Ile Ile Glu His Thr Asn
            260                 265                 270

cgg gtc atc ttc tta gaa gat gat gat atc gct gca gtg gct gat ggg      864
Arg Val Ile Phe Leu Glu Asp Asp Asp Ile Ala Ala Val Ala Asp Gly
        275                 280                 285

aaa ctc tcc att cac cga gtc aag cgc tca gct act gat gac ccc tcc      912
Lys Leu Ser Ile His Arg Val Lys Arg Ser Ala Thr Asp Asp Pro Ser
    290                 295                 300

cga gcc atc cag acc ttg cag atg gaa ctg cag caa ata atg aaa ggt      960
Arg Ala Ile Gln Thr Leu Gln Met Glu Leu Gln Gln Ile Met Lys Gly
305                 310                 315                 320

aac ttc agc gca ttt atg cag aag gag atc ttc gag cag cca gaa tca     1008
Asn Phe Ser Ala Phe Met Gln Lys Glu Ile Phe Glu Gln Pro Glu Ser
                325                 330                 335

gtt ttt aat acc atg aga ggt cgg gtg aat ttt gag acc aac aca gtg     1056
Val Phe Asn Thr Met Arg Gly Arg Val Asn Phe Glu Thr Asn Thr Val
            340                 345                 350

ctc ctg ggt ggc ttg aag gac cat ttg aaa gag atc cga cga tgc cga     1104
Leu Leu Gly Gly Leu Lys Asp His Leu Lys Glu Ile Arg Arg Cys Arg
        355                 360                 365
```

```
agg ctc att gtg att ggc tgt gga acc agc tac cat gcc gct gtg gct       1152
Arg Leu Ile Val Ile Gly Cys Gly Thr Ser Tyr His Ala Ala Val Ala
    370                 375                 380

aca cgg caa gtc tta gag gaa ctg acc gag ctg cct gtg atg gtt gaa       1200
Thr Arg Gln Val Leu Glu Glu Leu Thr Glu Leu Pro Val Met Val Glu
385                 390                 395                 400

ctt gcc agt gac ttt ctg gac agg aac aca cct gtg ttc agg gat gac       1248
Leu Ala Ser Asp Phe Leu Asp Arg Asn Thr Pro Val Phe Arg Asp Asp
                405                 410                 415

gtt tgc ttt ttc ata agc caa tca ggt gag act gca gac acg ctc ctg       1296
Val Cys Phe Phe Ile Ser Gln Ser Gly Glu Thr Ala Asp Thr Leu Leu
            420                 425                 430

gcg ctg cga tac tgt aag gat cga ggt gcg ctg acc gtg ggc atc acc       1344
Ala Leu Arg Tyr Cys Lys Asp Arg Gly Ala Leu Thr Val Gly Ile Thr
        435                 440                 445

aac acc gtg ggt agc tcc atc tcc cgg gag act gac tgt ggc gtc cac       1392
Asn Thr Val Gly Ser Ser Ile Ser Arg Glu Thr Asp Cys Gly Val His
    450                 455                 460

atc aac gca ggg ccc gag att ggg gtg gcc agc acc aag gcg tac acc       1440
Ile Asn Ala Gly Pro Glu Ile Gly Val Ala Ser Thr Lys Ala Tyr Thr
465                 470                 475                 480

agc cag ttc atc tct ctg gtg atg ttt ggt ttg atg atg tct gaa gat       1488
Ser Gln Phe Ile Ser Leu Val Met Phe Gly Leu Met Met Ser Glu Asp
                485                 490                 495

cga att tct cta cag aac agg aga caa gag atc atc cgt ggc ctc aga       1536
Arg Ile Ser Leu Gln Asn Arg Arg Gln Glu Ile Ile Arg Gly Leu Arg
            500                 505                 510

tct tta ccg gag ctg atc aaa gaa gtg ctg tcc ctg gat gag aag atc       1584
Ser Leu Pro Glu Leu Ile Lys Glu Val Leu Ser Leu Asp Glu Lys Ile
        515                 520                 525

cat gac ttg gcc ctg gag ctc tac aca caa agg tct ctc ctc gtg atg       1632
His Asp Leu Ala Leu Glu Leu Tyr Thr Gln Arg Ser Leu Leu Val Met
    530                 535                 540

gga cgg gga tat aac tat gcc aca tgt ctg gaa ggt gcc ttg aaa att       1680
Gly Arg Gly Tyr Asn Tyr Ala Thr Cys Leu Glu Gly Ala Leu Lys Ile
545                 550                 555                 560
```

```
aag gag ata acc tac atg cat tca gaa ggt atc cta gcc gga gag ctg      1728
Lys Glu Ile Thr Tyr Met His Ser Glu Gly Ile Leu Ala Gly Glu Leu
                565                 570                 575

aag cac ggg ccc ctt gct ctc gtc gac aag cag atg cca gtc atc atg      1776
Lys His Gly Pro Leu Ala Leu Val Asp Lys Gln Met Pro Val Ile Met
            580                 585                 590

gtc atc atg aag gat cct tgc ttt gcc aag tgc cag aat gcc ctg cag      1824
Val Ile Met Lys Asp Pro Cys Phe Ala Lys Cys Gln Asn Ala Leu Gln
        595                 600                 605

cag gtc act gcc cgc cag ggt cgc cca atc ata ctg tgt tcc aag gat      1872
Gln Val Thr Ala Arg Gln Gly Arg Pro Ile Ile Leu Cys Ser Lys Asp
    610                 615                 620

gac acc gag agc tcc aag ttt gca tat aaa acc att gaa ctt ccc cac      1920
Asp Thr Glu Ser Ser Lys Phe Ala Tyr Lys Thr Ile Glu Leu Pro His
625                 630                 635                 640

aca gtg gac tgt ctc cag ggt atc ctg agc gtg att cca ctc cag ctt      1968
Thr Val Asp Cys Leu Gln Gly Ile Leu Ser Val Ile Pro Leu Gln Leu
                645                 650                 655

ctg tcc ttc cac ctg gct gtc ctc cga ggt tat gat gtt gac ttc ccc      2016
Leu Ser Phe His Leu Ala Val Leu Arg Gly Tyr Asp Val Asp Phe Pro
            660                 665                 670

aga aac cta gcc aag tct gtc act gtg gaa tga                          2049
Arg Asn Leu Ala Lys Ser Val Thr Val Glu
        675                 680
```

```
<210>  5
<211>  682
<212>  PRT
<213>  Mus musculus

<400>  5

Met Cys Gly Ile Phe Ala Tyr Met Asn Tyr Arg Val Pro Lys Thr Arg
1               5                   10                  15

Lys Glu Ile Phe Glu Thr Leu Ile Arg Gly Leu Gln Arg Leu Glu Tyr
            20                  25                  30
```

```
Arg Gly Tyr Asp Ser Ala Gly Val Ala Ile Asp Gly Asn Asn His Glu
        35                  40                  45

Val Lys Glu Arg His Ile His Leu Val Lys Lys Arg Gly Lys Val Lys
    50                  55                  60

Ala Leu Asp Glu Glu Leu Tyr Lys Gln Asp Ser Met Asp Leu Lys Val
65                  70                  75                  80

Glu Phe Glu Thr His Phe Gly Ile Ala His Thr Arg Trp Ala Thr His
                85                  90                  95

Gly Val Pro Asn Ala Val Asn Ser His Pro Gln Arg Ser Asp Lys Asp
            100                 105                 110

Asn Glu Phe Val Val Ile His Asn Gly Ile Ile Thr Asn Tyr Lys Asp
        115                 120                 125

Leu Arg Lys Phe Leu Glu Ser Lys Gly Tyr Glu Phe Glu Ser Glu Thr
    130                 135                 140

Asp Thr Glu Thr Ile Ala Lys Leu Ile Lys Tyr Val Phe Asp Asn Arg
145                 150                 155                 160

Glu Thr Glu Asp Ile Thr Phe Ser Thr Leu Val Glu Arg Val Ile Gln
                165                 170                 175

Gln Leu Glu Gly Ala Phe Ala Leu Val Phe Lys Ser Ile His Tyr Pro
            180                 185                 190

Gly Glu Ala Val Ala Thr Arg Arg Gly Ser Pro Leu Leu Ile Gly Val
        195                 200                 205

Arg Ser Lys Tyr Lys Leu Ser Thr Glu Gln Ile Pro Val Leu Tyr Pro
    210                 215                 220
```

Thr Cys Asn Ile Glu Asn Val Lys Asn Ile Cys Lys Thr Arg Met Lys
225                 230                 235                 240

Arg Leu Asp Ser Ser Thr Cys Leu His Ala Val Gly Asp Lys Ala Val
                245                 250                 255

Glu Phe Phe Phe Ala Ser Asp Ala Ser Ala Ile Ile Glu His Thr Asn
            260                 265                 270

Arg Val Ile Phe Leu Glu Asp Asp Asp Ile Ala Ala Val Ala Asp Gly
        275                 280                 285

Lys Leu Ser Ile His Arg Val Lys Arg Ser Ala Thr Asp Asp Pro Ser
    290                 295                 300

Arg Ala Ile Gln Thr Leu Gln Met Glu Leu Gln Gln Ile Met Lys Gly
305                 310                 315                 320

Asn Phe Ser Ala Phe Met Gln Lys Glu Ile Phe Glu Gln Pro Glu Ser
                325                 330                 335

Val Phe Asn Thr Met Arg Gly Arg Val Asn Phe Glu Thr Asn Thr Val
            340                 345                 350

Leu Leu Gly Gly Leu Lys Asp His Leu Lys Glu Ile Arg Arg Cys Arg
        355                 360                 365

Arg Leu Ile Val Ile Gly Cys Gly Thr Ser Tyr His Ala Ala Val Ala
    370                 375                 380

Thr Arg Gln Val Leu Glu Glu Leu Thr Glu Leu Pro Val Met Val Glu
385                 390                 395                 400

Leu Ala Ser Asp Phe Leu Asp Arg Asn Thr Pro Val Phe Arg Asp Asp
                405                 410                 415

```
Val Cys Phe Phe Ile Ser Gln Ser Gly Glu Thr Ala Asp Thr Leu Leu
            420                 425                 430

Ala Leu Arg Tyr Cys Lys Asp Arg Gly Ala Leu Thr Val Gly Ile Thr
        435                 440                 445

Asn Thr Val Gly Ser Ser Ile Ser Arg Glu Thr Asp Cys Gly Val His
    450                 455                 460

Ile Asn Ala Gly Pro Glu Ile Gly Val Ala Ser Thr Lys Ala Tyr Thr
465                 470                 475                 480

Ser Gln Phe Ile Ser Leu Val Met Phe Gly Leu Met Met Ser Glu Asp
                485                 490                 495

Arg Ile Ser Leu Gln Asn Arg Arg Gln Glu Ile Ile Arg Gly Leu Arg
            500                 505                 510

Ser Leu Pro Glu Leu Ile Lys Glu Val Leu Ser Leu Asp Glu Lys Ile
        515                 520                 525

His Asp Leu Ala Leu Glu Leu Tyr Thr Gln Arg Ser Leu Leu Val Met
    530                 535                 540

Gly Arg Gly Tyr Asn Tyr Ala Thr Cys Leu Glu Gly Ala Leu Lys Ile
545                 550                 555                 560

Lys Glu Ile Thr Tyr Met His Ser Glu Gly Ile Leu Ala Gly Glu Leu
                565                 570                 575

Lys His Gly Pro Leu Ala Leu Val Asp Lys Gln Met Pro Val Ile Met
            580                 585                 590

Val Ile Met Lys Asp Pro Cys Phe Ala Lys Cys Gln Asn Ala Leu Gln
        595                 600                 605
```

```
Gln Val Thr Ala Arg Gln Gly Arg Pro Ile Ile Leu Cys Ser Lys Asp
    610                 615                 620

Asp Thr Glu Ser Ser Lys Phe Ala Tyr Lys Thr Ile Glu Leu Pro His
625                 630                 635                 640

Thr Val Asp Cys Leu Gln Gly Ile Leu Ser Val Ile Pro Leu Gln Leu
                645                 650                 655

Leu Ser Phe His Leu Ala Val Leu Arg Gly Tyr Asp Val Asp Phe Pro
            660                 665                 670

Arg Asn Leu Ala Lys Ser Val Thr Val Glu
        675                 680
```